# EUROPEAN PATENT SPECIFICATION

(11) **EP 2 551 356 B1**
(45) Date of publication and mention of the grant of the patent: **14.06.2017**
(21) Application number: 11759408.5
(22) Date of filing: 23.03.2011
(51) Int. Cl.: C12Q 1/68, C12N 15/09

(54) **METHOD FOR DETECTING TARGET BASE SEQUENCE USING COMPETITIVE PRIMER**
VERFAHREN ZUR ERKENNUNG VON ZIELBASISSEQUENZEN MITHILFE KONKURRIERENDER PRIMER
MÉTHODE PERMETTANT DE DÉTECTER UNE SÉQUENCE DE BASES CIBLE À L'AIDE D'UNE AMORCE COMPÉTITIVE

(30) Priority: 24.03.2010 JP 2010068490
(43) Date of publication of application: 30.01.2013
(73) Proprietor: Toppan Printing Co., Ltd., Tokyo 110-8560 (JP)
(72) Inventor: MAKINO, Yoichi, Tokyo 110-8560 (JP); YAMANE, Akio, Tokyo 110-8560 (JP); NAKAYAMA, Masato, Tokyo 110-8560 (JP)
(74) Representative: Bailey, Jennifer Ann
(86) International application number: PCT/JP2011/056892
(87) International publication number: WO 2011/118603

(56) References cited:
- EP-A1- 1 241 266
- EP-A2- 0 333 465
- EP-A2- 1 061 135
- WO-A1-01/42498
- JP-A- 2001 057 892
- JP-A- 2001 286 300
- JP-A- 2002 171 986
- JP-A- 2005 027 518
- JP-A- 2005 287 499
- JP-A- 2009 247 230
- GIBBS R A ET AL: "Detection of single DNA base differences by competitive oligonucleotide priming", NUCLEIC ACIDS RESEARCH, OXFORD UNIVERSITY PRESS, SURREY, GB, vol. 17, no. 7, 1 January 1989 (1989-01-01), pages 2437-2448, XP009171762, ISSN: 0305-1048
- KURATA, S. ET AL.: 'Fluorescent quenching-based quantitative detection of specific DNA/RNA using a BODIPYO FL-labeled probe or primer. abstr e34' NUCLEIC ACIDS RES. vol. 29, no. 6, 2001, pages 1 - 5, XP002376104
- MERRYWEATHER-CLARKE A T ET AL: "A RAPID NON-INVASIVE METHOD FOR THE DETECTION OF THE HAEMOCHROMATOSIS C282Y MUTATION", BRITISH JOURNAL OF HAEMATOLOGY, WILEY-BLACKWELL PUBLISHING LTD, GB, vol. 99, 1 January 1997 (1997-01-01), pages 460-463, XP002906810, ISSN: 0007-1048, DOI: 10.1046/J.1365-2141.1997.4193224.X
- DONOHOE G G ET AL: "RAPID SINGLE-TUBE SCREENING OF THE C282Y HEMOCHROMATOSIS MUTATION BY REAL-TIME MULTIPLEX ALLELE-SPECIFIC PCR WITHOUT FLUORESCENT PROBES", CLINICAL CHEMISTRY, AMERICAN ASSOCIATION FOR CLINICAL CHEMISTRY, WASHINGTON, DC, vol. 46, no. 10, 1 January 2000 (2000-01-01), pages 1540-1547, XP002906811, ISSN: 0009-9147
- CASADO-DIAZ ET AL: "Individual single tube genotyping and DNA pooling by allele-specific PCR to uncover associations of polymorphisms with complex diseases", CLINICA CHIMICA ACTA, ELSEVIER BV, AMSTERDAM, NL, vol. 376, no. 1-2, 13 December 2006 (2006-12-13), pages 155-162, XP005802751, ISSN: 0009-8981, DOI: 10.1016/J.CCA.2006.08.014

## Description

### TECHNICAL FIELD

The present invention relates to a method of detecting a target base sequence having a polymorphic base, and a kit for use in the above-mentioned method. In more detail, it relates to a method of detecting a target base sequence with high precision by allowing primers to compete with each other, and a kit for use in the above-mentioned method.

Priority is claimed on Japanese Patent Application No. 2010-68490, filed March 24,2010.

### BACKGROUND ART

Recently the worldwide human genome analysis has elucidated the sequences of 3.1 billion base pairs, and revealed that the number of human genes is approximately 30,000 to 40,000.

There are differences in the base sequences between individual humans. The occurrence of such a difference with one or more percent frequency in a certain population group is referred to as a gene polymorphism. In particular, SNP (Single Nucleotide Polymorphism), which is a difference of only a single base in the sequence of a gene, has been suggested to be associated with various types of diseases. For example, genetic diseases of humans are attributed to a single base difference in a gene. In addition, lifestyle-related diseases and cancer are thought to be influenced by single base differences of a plurality of genes.

Accordingly, the SNP analysis is considered to be quite effective for the development of medicines, including the search for the target of drug discovery and the prediction of side effects. For this reason, the SNP analysis has been pushed forward as a huge worldwide project.

One of the reasons why there are individual differences in the degree of drug effects and side effects is considered to be individual differences in a group of enzymes related to the metabolism of the drug. It has been being revealed these days that such differences are also due to tiny differences in genes.

Therefore, a method has been considered in which gene(s) of a patient is(are) analyzed in advance to select an optimum drug before administering it to the patient. Furthermore, the importance of such genetic diagnosis is rapidly increasing not only for single-gene diseases but also for multi-factor diseases.

In addition, the effect of a drug targetting a pathogenic bacterium or a virus is sometimes different per each individual even within a same species. It is often due to tiny difference(s) of gene(s) per each individual. In the diagnosis of gene(s) of such a pathogenic bacterium, a virus, or like foreign factor, it is surely expected that the number of the test subjects will increase in the future.

In this way, it is important for the medical treatment in the post genomic era to be able to detect a tiny difference of a gene, particularly a single base difference, of human and pathogenic microbes, and it is expected that such importance will increase in the future.

So far, various types of methods to detect a tiny difference, particularly a single base difference in a base sequence, have been investigated (refer to Non-patent Documents 1 to 2).

However, in order to carry out the detection in a practical level, all points including the low cost, the easiness and convenience of the method, the shortness of the detection time, and the accuracy of the detection result, have to be excellent. However, until now, no method has been known that can satisfy all the above-mentioned requirements.

Upon the detection of a tiny difference, particularly a single-base difference of a gene, it is usual that only a small amount of fragments of the target gene is contained in each sample. In this case, it is neessary to previously amplify the target gene by some means. Such means to amplify the gene can be exemplified by the PCR (Polymerase Chain Reaction) method.

Generally speaking, the detection of a single-base difference of a target gene requires two stage processes: a stage of gene amplification, and a stage of checking the single-base difference of the amplified gene (refer to Non-patent Document 2). However, in such a method requiring two stage processes, the manipulation is complicated since there are a plurality of processes.

Some methods have been reported so as to improve such a complicated situation of two stage processes, such as, for example, the TaqMan method using a probe that is attached to a fluorophore and a quencher (refer to Non-patent Document 3), and the MALDI-TOF/MS method which employs DNA mass spectrometry using a mass spectrometer (refer to Non-patent Document 4). In addition, as a method not requiring gene amplification, the Invader method has been reported which uses an enzyme that can recognize the DNA structure to cleave it (refer to Non-patent Document 5). However, these methods are still expensive to implement and these probes are complicated to design.

On the other hand, a method has been reported which simultaneously conducts gene amplification and single base identification (refer to Non-patent Document 6). This method utilizes a phenomenon in which the occurrence of an extension reaction of a DNA polymerase depends on whether or not the 3' end of a primer is complementary (hereunder, called match) to the template DNA in the sample. In other words, in the case where one of a primer set consisting of a primer pair for use in the PCR reaction is designed so that the 3' end of the primer has a base complementary to the single base polymorphism, and if the primer is completely matched to the template, the extension reaction occurs and an amplification reaction with the other primer is brought about. However, if there is a single-base mismatch between the primer and the template, the extension reaction from the primer hardly occurs and an amplification reaction with the other primer also hardly occurs. In this way, the single base can be identified by referring to the amount of the amplification product from the amplification reaction. According to this method, there is no need of carrying out additional manipulation after the amplification reaction to identify a single base.

However, in this method, the reaction is suspecible to the reaction conditions, for example, the amount of the template, the temperature, the amount of the primer, the concentration of dNTPs serving as the substrate of the reaction, and the like. For this reason, it is not always easy to obtain reproducible data.

As a different method, a method has been investigated in which, for example, an artificial mutation (a base that is a mismatch against the template) is introduced in a vicinity of the 3' end of a primer (refer to Non-patent Document 7). However, even with this method, a certain level of labor is necessary for the optimization of the primer and the identification precision is sometimes influenced by the quality of the sample.

In order to solve these problems, some methods have been proposed in which at least two types of primers labeled with a fluorophore or the like are reacted in a competitive manner (refer to Patent Documents 1 to 4).

The detection method as proposed in Patent Documents 1 to 3 utilizing the phenomenon such that, having the 3' end or the vicinity of the end of the primer correspond to the position of the base to be examined, the extension reaction occurs when it matches to the base of the target nucleic acid in a sample, while the extension reaction hardly occurs when it is a mismatch, is referred to as allele-specific PCR (ASP-PCR).

It has been known that, upon the allele-specific PCR, the base identification precision is higher in the case where two or more allele-specific primers are allowed to compete with each other, than in the case where the respective allele-specific primers are separately amplified (refer to Non-patent Document 8). This is because, in a case where an allele differing from the allele to be detected is present, the primer that can match to the different allele preferentially binds to the target base sequence, thus making it possible to suppress the nonspecific extension reaction of the primer to be detected. Furthermore, since the extension from the primer that can match to the different allele can be efficiently progressed so that the materials necessary for the extension reaction are consumed, it is also possible to suppress the formation of a primer dimer of the primer that is a mismatch against the allele to be detected. Such a method in which primers are competitively reacted in this way is referred to as Competitive Oligonucleotide Priming (COP). COP is used in Patent Documents 1 to 4.

The method proposed in Patent Document 1 utilizes a tendency in which a competitive primer having fewer number of mismatch bases, out of a plurality of competitive primers having different numbers of mismatch bases against the target nucleic acid, is more likely to form a double strand with the target nucleic acid. It is an excellent method for the case of detecting a single base mutation in the target nucleic acid in an easy and convenient way.

However, in this method, false positives might occur during the SNP analysis. This can be attributed to the insufficiency in the capability to suppress the extension from the competitive primer that has a larger number of mismatch bases, since the competitive primer has been designed only by focusing on the single base mutation in the target nucleic acid.

It is important for the allele-specific primer for use in the allele-specific PCR to be stable in the vicinity of the 3' end of the double strand formed with the target nucleic acid. The base identifiability can be much improved by setting so that base(s) other than the base to be identified would not be complementary to the target nucleic acid in the vicinity of the 3' end.

In the SNP detection method proposed in Patent Document 2, for the purpose of reducing the false positive problem of Patent Document 1, a base to identify SNP is introduced in the 3' end of the competitive primer, and furthermore artificial mutation(s) is(are) introduced in one or more of the second to fifth bases from the 3' end.

In addition, in the method of detecting a base polymorphism proposed in Patent Document 3, artificial mutation(s) is(are) introduced in position(s) from the first to fifth bases from the 3' end of the competitive primer.

The methods proposed in Patent Documents 2 and 3 are to introduce the same mutation(s) in the same position(s) between competitive primers, and utilize the phenomenon that the extension reaction is efficiently progressed when the vicinity of the 3' end of the primer can match to the target nucleic acid while the extension efficiency drops as the number of mismatch bases increases. Moreover, the fact that the double strand of the primer and the target nucleic acid becomes unstable as a whole as the number of mismatch bases increases, is also associated with the reduction of the extension efficiency.

The method of detecting a base polymorphism proposed in Patent Document 4 states that different mutations are introduced in the same position(s) between competitive primers. EP 0 333 465 describes competitive priming with primers having mismatch sites and a common reverse primer to target nucleic acid in a sample. EP 1 241 266 describes a method for detecting target nucleic acid having a polymorphism. Primers with mismatches are used. Gibbs et al (Nucleic acid research, Vol. 17, No. 1, 1989, 2437-2448) describes the use of primers with mismatches for DNA synthesis.

### Prior Art Documents

### [Patent Documents]

Patent Document 1: Japanese Patent (Granted) Publication No. 2760553
Patent Document 2: Japanese Laid-Open Patent Application No. 2003-52372
Patent Document 3: Japanese Laid-Open Patent Application No. 2005-287499
Patent Document 4: Japanese Patent (Granted) Publication No. 4228041

### [Non-patent Documents]

Non-patent Document 1: Landegren, Laboratory protocols for mutation detection, Oxford University Press, 1996
Non-patent Document 2: Ahmadian et. al. Biotechniques, Vol. 32, pp. 1122-1137, 2002
Non-patent Document 3: Livak et. al. PCR Methods Appl., Vol. 5, pp. 357-362, 1995
Non-patent Document 4: Griffin et. al. Trends Biotechnol., Vol. 18, pp. 77-84, 2000
Non-patent Document 5: Ryan et. al. Molecular diagnosis, Vol. 4, pp. 135-144, 1999
Non-patent Document 6: Okayama et. al. J. Lab. Clin. Med., Vol. 114, pp. 105-113,1989
Non-patent Document 7: Newton et. al. Nucleic Acids Res., Vol. 17, pp. 2503-2516, 1989
Non-patent Document 8: Myakishev et. al. Genome Res., Vol. 11, pp. 163-169, 2001
Non-patent Document 9: Shinozuka et. al. J. Chem. Soc., Chem. Commun., Vol. 10, pp. 1377-1378, 1994

### DISCLOSURE OF INVENTION

### PROBLEMS TO BE SOLVED BY THE INVENTION

However, even with these methods, there is still an insufficiency in the capability to suppress the extension from the competitive primer that has a mismatch against the template in the vicinity of the 3' end.

The present invention addresses the above-mentioned situations, with an object of providing: a method of detecting a target base sequence with high identification precision while offering equivalent easiness and convenience to those of conventional methods; and a kit for use in the above-mentioned method.

### MEANS TO SOLVE THE PROBLEMS

In order to solve the above problems, the inventors of the present invention have conducted intensive studies. As a result, they have discovered that these problems can be solved by taking into consideration of the position(s) of the mutation(s) to be introduced into the competitive primer.

According to a first aspect, the present invention provides:
a method of detecting a target base sequence having a polymorphic base comprising
   (a) a step of adding the following primers to a nucleic acid sample having a target nucleic acid that comprises a base sequence including the target base sequence:
      at least one type of detection primer that is complementary to said target base sequence,
      at least one type of competitive primer that is complementary to said target base sequence as well as being capable of annealing to a target nucleic acid, in a competitive manner against said detection primer, and
      at least one type of common primer,
   (b) a step of annealing said detection primer and said competitive primer to said target nucleic acid in a competitive manner with use of the target base sequence having the polymorphic base in said nucleic acid sample as a template, thereby causing an extension reaction to synthesize an extension product A,
   (c) a step of annealing said common primer to said extension product A obtained in said step (b) or in the following step (d), thereby causing an extension reaction to synthesize an extension product B,
   (d) a step of annealing said detection primer or said competitive primer to the extension product B obtained in said step (c), thereby synthesizing the extension product A, and
   (e) a step of detecting the extension product A or the extension product B, wherein
   said detection primer has a match base that is complementary to the polymorphic base of the target sequence, and has at least one first mismatch base that is not complementary to a base of the target sequence other than the polymorphic base;
   said competitive primer has a mismatch base that is not complementary to the polymorphic base of the target sequence, and has at least one first mismatch base and optionally at least one second mismatch base that are not complementary to a base of the target sequence other than the polymorphic base;
   the position of the at least one first mismatch base of said detection primer is different from the position of the at least one first mismatch base of said competitive primer that is not complementary to the base of the target sequence other than the polymorphic base;
   the detection primer has the match base that is complementary to the polymorphic base of the target sequence, at the 3' end or the second base from the 3' end;
   said common primer is capable of making a primer pair with said detection primer or said competitive primer to amplify said target nucleic acid;
   a difference in a chain length between said competitive primer and said detection primer is within 16 bases;
   when present, the second mismatch base of said competitive primer is located 7 bases or more away from the 3' end to the 5' side.

Optional features are specified by the dependent claims.

In the method of detecting a target base sequence, in the detection primer and the competitive primer, the mismatch base that is not complementary to a base other than the polymorphic base may be located within 17 bases from the match base that is complementary to the polymorphic base in the case of the detection primer, and from the mismatch base that is not complementary to the polymorphic base in the case of the competitive primer, and the position of the mismatch base of each primer may be different.

In the method of detecting a target base sequence, in the detection primer and the competitive primer, a first mismatch base may be located within 6 bases from the 3' end, a second mismatch base may be located 7 bases or more away from the 3' end to the 5' side, , and the second mismatch base of the detection primer and the second mismatch base of the competitive primer may be different from each other.

In the method of detecting a target base sequence, the position of the second mismatch base may be the same for both the detection primer and the competitive primer.

In the method of detecting a target base sequence, the steps (b) to (d) may be steps performed by any one selected from a group consisting of PCR, LAMP, NASBA, ICAN, TRC, SDA, TMA, SMAP, RPA, and HDA.

In the method of detecting a target base sequence, at least one of the detection primer, the competitive primer, and the common primer may be labeled.

In the method of detecting a target base sequence, a labeling substance for use in the labeling may be at least one selected from a group consisting of a fluorophore and an energy absorbing material.

In the method of detecting a target base sequence , the detection primer and the competitive primer may be respectively labeled with different types of labeling substances, and the step (e) may be a step of separately detecting the extension product from the detection primer and the extension product from the competitive primer.

In the method of detecting a target base sequence, the step (e) may be a step to be performed simultaneously with the steps (b) to (d), as well as being a step of detecting a state where the extension product from a labeled primer forms a double strand.

In the method of detecting a target base sequence, the step (e) may be a step to be performed after the step (d), as well as being a step of performing the detection with use of a melting curve or an amplification curve of the extension product.

In the method of detecting a target base sequence, the step (e) may be a step of performing the detection through use of a QP (Quenching Probe/Primer) method.

According to a second aspect, the present invention provides a kit for use in the method of detecting a target base sequence having a polymorphic base comprising at least one type of detection primer that is complementary to said target base sequence,
at least one type of competitive primer that is complementary to said target base sequence as well as being capable of annealing to target nucleic acid in a competitive manner against said detection primer, and
at least one type of common primer, wherein
said detection primer has a match base that is complementary to the polymorphic base of the target sequence, and has at least one first mismatch base that is not complementary to a base of the target sequence other than the polymorphic base of said target sequence;
said competitive primer has a mismatch base that is not complementary to the polymorphic base of the target sequence, and has at least one first mismatch base and optionally at least one second mismatch base that are not complementary to a base of the target sequence other than the polymorphic base;
the position of the at least one first mismatch base of said detection primer is different from the position of the at least one first mismatch base of said competitive primer that is not complementary to the base of the target sequence other than the polymorphic base;
the detection primer has the match base that is complementary to the polymorphic base of the target sequence, at the 3' end or the second base from the 3' end;
said common primer is capable of making a primer pair with said detection primer or said competitive primer to amplify said target nucleic acid;
a difference in a chain length between said competitive primer and said detection primer is within 16 bases;
when present, the second mismatch base of said competitive primer is located 7 bases or more away from the 3' end to the 5' side.

The method may include (a) a step of adding the detection primer and the competitive primer to a nucleic acid sample having the target nucleic acid, (b) a step of annealing the detection primer and the competitive primer to the target nucleic acid in a competitive manner with use of the target base sequence having the polymorphic base in the nucleic acid sample as a template, thereby causing an extension reaction to synthesize an extension product A, (c) a step of annealing the common primer to the extension product A obtained in the step (b) or in the following step (d), thereby causing an extension reaction to synthesize an extension product B, (d) a step of annealing the detection primer or the competitive primer to the extension product B obtained in the step (c), thereby synthesizing the extension product A, and (e) a step of detecting the extension product A or the extension product B.

### EFFECTS OF THE INVENTION

According to the method of detecting a target base sequence of the present invention, a single nucleotide polymorphism and a single-base somatic mutation can be identified with high precision, while offering equivalent easiness and convenience to those of conventional methods.

In addition, according to the target base sequence detection kit of the present invention, the method of detecting a target base sequence of the present invention can be more easily and conveniently conducted.

### BRIEF DESCRIPTION OF THE DRAWINGS

FIG. 1 is a schematic diagram showing one aspect of a conventional method of detecting a target base sequence
FIG. 2 is a schematic diagram showing another aspect of the conventional method of detecting a target base sequence
FIG. 3 is a schematic diagram showing one aspect of the method of detecting a target base sequence of the present invention.
FIG. 4 is a schematic diagram showing another aspect of the method of detecting a target base sequence of the present invention.
FIG. 5 is a schematic diagram showing yet another aspect of the method of detecting a target base sequence of the present invention.
FIG. 6 is a schematic diagram showing even yet another aspect of the method of detecting a target base sequence of the present invention.
FIG. 7A is a graph showing the amount of products extended from the primers [1] and [2] per each round in the case where these primers have been competitively reacted.
FIG. 7B is a graph showing the amount of products extended from the primers [1] and [2] per each round in the case where these primers have been competitively reacted.
FIG. 8A is a graph showing the amount of products extended from the primers [1] and [3] per each round in the case where these primers have been competitively reacted.
FIG. 8B is a graph showing the amount of products extended from the primers [1] and [3] per each round in the case where these primers have been competitively reacted.
FIG. 9A is a graph showing the amount of products extended from the primers [1] and [4] per each round in the case where these primers have been competitively reacted.
FIG. 9B is a graph showing the amount of products extended from the primers [1] and [4] per each round in the case where these primers have been competitively reacted.
FIG. 10 is a schematic diagram showing the method to discriminate the single stranded state and the double stranded state. Open circle; Acridine, Open diamond; Fluorescein
FIG. 11 shows the results of Comparative Example 1 expressed by negative first differential curves of the melting curves thereof.
FIG. 12 shows the results of Comparative Example 2 expressed by negative first differential curves of the melting curves thereof.
FIG. 13 shows the results of Example 1 expressed by negative first differential curves of the melting curves thereof.
FIG. 14 shows the results of Example 2 expressed by negative first differential curves of the melting curves thereof.
FIG. 15 shows the results of Example 3 expressed by negative first differential curves of the melting curves thereof.
FIG. 16 shows the results of Example 4 expressed by negative first differential curves of the melting curves thereof.
FIG. 17 shows the results of Example 5 expressed by negative first differential curves of the melting curves thereof.
FIG. 18 shows the results of Example 6 expressed by negative first differential curves of the melting curves thereof.
FIG. 19 shows the results of Example 7 expressed by negative first differential curves of the melting curves thereof.
FIG. 20 shows the results of Example 8 expressed by negative first differential curves of the melting curves thereof.
FIG. 21 shows the results of Example 9 expressed by negative first differential curves of the melting curves thereof.
FIG. 22 shows the results of Example 10 expressed by negative first differential curves of the melting curves thereof.
FIG. 23 shows the results of Example 11 expressed by negative first differential curves of the melting curves thereof.
FIG. 24 shows the results of Example 12 expressed by negative first differential curves of the melting curves thereof.
FIG. 25 shows the results of Example 13 expressed by negative first differential curves of the melting curves thereof.
FIG. 26 shows the results of Example 14 expressed by negative first differential curves of the melting curves thereof.
FIG. 27 shows the results of Example 15 expressed by negative first differential curves of the melting curves thereof.
FIG. 28 shows the results of Example 16 expressed by negative first differential curves of the melting curves thereof.
FIG. 29 shows the results of Example 17 expressed by negative first differential curves of the melting curves thereof.
FIG. 30 shows the results of Example 18 expressed by negative first differential curves of the melting curves thereof.
FIG. 31 shows the results of Examples 19 to 27 expressed by amplification curves.
FIG. 32 shows the results of Examples 28 to 35 expressed by amplification curves.

### EMBODIMENTS OF THE INVENTION

The method of detecting a target base sequence of the present invention is a method of detecting a target nucleic acid having a polymorphic base.

In the present invention, the term "target base sequence" refers to a base sequence that has a polymorphic base.

In the present invention, the phrase "detecting a target base sequence" refers to detecting whether or not the base sequence of a nucleic acid contained in a nucleic acid sample has an identical base sequence to a known base sequence.

In the description and the claims of this application, a nucleic acid which includes a polymorphism serving as an identification target is referred to as the "target nucleic acid".

The target nucleic acid is not specifically limited as long as the target nucleic acid consists of a base sequence that includes a polymorphic base, and the base sequence thereof has been elucidated to a degree that enables to design a primer which can anneal to the target nucleic acid.

In addition, the polymorphic base of the target base sequence may be either an inherent polymorphism such as SNP or an acquired polymorphism such as somatic mutation, as long as it is a polymorphism of a single base.

The term "SNP (single nucleotide polymorphism)" is defined as a single base variation of a genomic base sequence between individuals within a same biological species, with 1% or more frequency of the whole group population.

On the other hand, the term "somatic mutation" means an acquired variation of a gene between cells that occurs within a same individual. In addition, the term "mutation site" means a site in which certain base(s) in a sequence is(are) changed. Such a mutation in a base sequence does not only consist of a substitution of a single base, but also may include cases of substitution, deletion, or insertion of a plurality of bases.

The polymorphism serving as the target of detection can be exemplified by polymorphisms of genes associated with various types of diseases such as genetic diseases, lifestyle-related diseases, and cancer, and genes associated with drug metabolism. The present invention is particularly suitably used for the detection of a gene polymorphism (1173C>T) in the position 1173 of the intron 1 region of the vitamin K epoxide reductase complex 1 (*VKORC1*)*,* which is a gene related to the optimum dose of warfarin, as well as for the detection of a mutation of the 12^{th} or 13^{th} codon in the *K-Ras* cancer gene.

In the present invention, the term "detection primer" refers to a primer to detect the target base sequence.

The detection primer has a match base that is complementary to the polymorphic base, and has at least one first mismatch base that is not complementary to a base other than the polymorphic base of the target base sequence.

The number of mismatch base(s) held by the detection primer may be one or two or more. The number is preferably from 1 to 5, more preferably from 1 to 3, and particularly preferably 1 or 2.

In the present invention, the term "competitive primer" refers to a primer which can anneal to a region including the polymorphic base in the target nucleic acid, in a competitive manner against the detection primer, and which has a mismatch base that is not complementary to the polymorphic base. Since the competitive primer has a mismatch base that is not complementary to the polymorphic base, it does not form a base pair with the polymorphic base in the target nucleic acid when it anneals to the target nucleic acid. Moreover, the competitive primer has at least one mismatch base that is not complementary to a base other than the polymorphic base of the target base sequence, in addition to the mismatch base that is not complementary to the polymorphic base.

The number of mismatch base(s) held by the competitive primer may be one or two or more. The number is preferably from 1 to 5, more preferably from 1 to 3, and particularly preferably 1 or 2.

Note that, in the present invention, the term "match" means a state in which a pair of DNA bases constituting a double strand forms a Watson-Crick type base pair, and the term "mismatch" means a state in which a Watson-Crick type base pair is not formed. The term "Watson-Crick type base pair" refers to a linking via hydrogen bonds between two polynucleotide molecules of deoxyribonucleic acids by having a pair of adenine (A) and thymine (T) (or uracil (U)) and a pair of guanine (G) and cytosine (C). The mismatch base may be either naturally derived or artificially created, although the base type of the mismatch base has to be different from that of the template.

The position of the at least one mismatch base of the detection primer is different from the position(s) of the mismatch base(s) of the competitive primer. Accordingly, there are at least 3 bases differences in the base sequence between the detection primer and the competitive primer.

Here, the term "position(s) of the mismatch base(s)" refers to the position(s) relative to the base that corresponds to the polymorphic base in the primer, when the template and the primer form a double strand.

By allowing the both primers to anneal to the target nucleic acid in a competitive manner, the precision to detect the polymorphic base can be improved. This is because: in a case where an allele differing from the allele to be detected (the target nucleic acid in the present invention) is present, the competitive primer will preferentially bind to the different allele when a primer that can detect the target allele is used as a detection primer and a primer that can detect the different allele is used as a competitive primer; thus making it possible to suppress the nonspecific reaction of the detection primer. In addition, since the extension from the competitive primer can be efficiently progressed due to the difference in the stability in the vicinity of the 3' end, the materials necessary for the extension reaction are consumed. This makes it possible to greatly suppress the nonspecific amplification from the detection primer.

In the present invention, the detection primer and the competitive primer are complementary to the target base sequence. Here, in the present invention, the phrase "complementary" means that an oligonucleotide has a base sequence capable of forming a double stranded state with a target nucleic acid that has a specific sequence under a reaction condition for an extension reaction. It is not necessary to be completely complementary, meaning that several mismatch base pairs may be included.

In addition, the detection primer and the competitive primer may be annealed to respectively different regions, as long as these are within the target base sequence. From the point to anneal to the target nucleic acid in a competitive manner, it is preferable to design both primers so that the competitive primer can anneal to the target nucleic acid in the same region as the region to which the detection primer can anneal.

In the present invention, the detection primer has a base that is complementary to the polymorphic base, at the 3' end or the second base from the 3' end. From the point to anneal to the target nucleic acid in a competitive manner, it is preferable that competitive primer has a base that is not complementary to the polymorphic base, at the 3' end or the second base from the 3' end.

For example, assuming that the target base sequence is 5'-ATGCATGC-3' and A at the fifth base from the 5' end serves as the polymorphic base, the sequence in the vicinity of the 3' end of the detection primer for detecting this polymorphic base A can be 5'-GCAT-3' or 5'-GCATG-3'. In this case, the sequence in the vicinity of the 3' end of the competitive primer can be 5'-GCAG-3' or 5'-GCAGG. The above-mentioned example is only an example, and the mismatch base can be selected from other three types of bases differing from the type of the base serving as the match base. The position of the detection primer to identify the base of the template is preferably the 3' end or the second base from the 3' end, although it may be away from the 3' end.

The base type of the base to be a mismatch against the polymorphic base in the base sequence of the competitive primer is preferably a base type that can match to another genotype of polymorphism differing from that of the target base sequence. For example, in the case of detecting the mutant type of a polymorphism whose wild-type is C and mutant type is A, and in a case where a base sequence including the polymorphic base A is adopted as the target base sequence, it is possible to select any one of A, G, and C for use as the base that is not complementary to the polymorphic base A. However, it is preferable to select G which is complementary to the wild-type.

In the present invention, it is possible to use one or two or more types of competitive primer(s). For example, as with *K-ras* that will be described later, in the case where the polymorphism includes a plurality of genotypes, it is preferable to use a plurality of types of competitive primers which respectively match to respective genotypes differing from the genotype of the detection target.

In the present invention, the term "common primer" refers to a primer which is capable of making a pair with the detection primer or the competitive primer to amplify the target nucleic acid, has a sequence that can match to 10 to 30 bases on the 3' end side of the extension product from the detection primer or the competitive primer, and has a capability to extend in a PCR reaction with use of the extension product from the detection primer or the competitive primer, as a template. In the present invention, it is possible to use two or more types of common primers, and the two or more types of common primers may have a competitive relationship each other. Such two or more types of common primers having a competitive relationship may have a polymorphic base sequence.

It is preferable that the mismatch base of the detection primer and the competitive primer that is not complementary to a base other than the polymorphic base is located within 17 bases, and more preferably within 8 bases, from the match base that is complementary to the polymorphic base in the case of the detection primer, and from the mismatch base that is not complementary to the polymorphic base in the case of the competitive primer.

The detection primer and the competitive primer may have a plurality of mismatch bases respectively. If the detection primer and the competitive primer have two mismatch bases respectively, it is preferable in the detection primer and the competitive primer that the first mismatch base is located within 6 bases from the 3' end, and the second mismatch base is located 7 bases or more away from the 3' end to the 5' side.

The position of the first mismatch base of the detection primer is different from the position of the first mismatch base of the competitive primer.

In addition, the second mismatch base of the detection primer and the second mismatch base of the competitive primer may be different from each other in the position or in the base type.

In other words, if the positions are the same, the base types may be different.

In the present invention, the phrase "the second mismatch base of the detection primer and the second mismatch base of the competitive primer are different" specifically means a case where their positions are different, or a case where the both positions are the same and the both base types are different.

In the present invention, the position of the second mismatch base may be the same for both the detection primer and the competitive primer.

It is preferable in the detection primer and the competitive primer that the second mismatch base is located 7 bases or more away from the 3' end, and more preferably 9 bases or more away from the 3' end.

In addition, it is preferable to arrange the second mismatch base around the center of the primer, from the point of improving the efficiency of the amplification reaction from the detection primer. For example, when using a primer of 20 to 25 bases, it is preferable to locate the second mismatch base at the 7th to the 15th base from the 3' end, and more preferably at the 9th to the 15th base from the 3' end.

The lengths of the detection primer and the competitive primer might have influences on the identifiability and the reactivity. For example, a primer having a longer chain length tends to more preferentially anneal to the target nucleic acid. Accordingly, in order to detect the target base sequence more precisely, the difference in the chain length between the competitive primer and the detection primer is within 16 bases, preferably within 2 bases, and particularly preferably within 1 base.

For example, if there is a difference in the chain length and the chain length of the competitive primer is longer, then the competitive primer can anneal more preferentially than the detection primer, and the detection primer may be inhibited from annealing. Conversely, if the chain length of the detection primer is longer, then the detection primer can anneal more preferentially than the competitive primer, and the competitive primer may be inhibited from annealing. For this reason, it is preferable that the detection primer and the competitive primer have equivalent chain lengths.

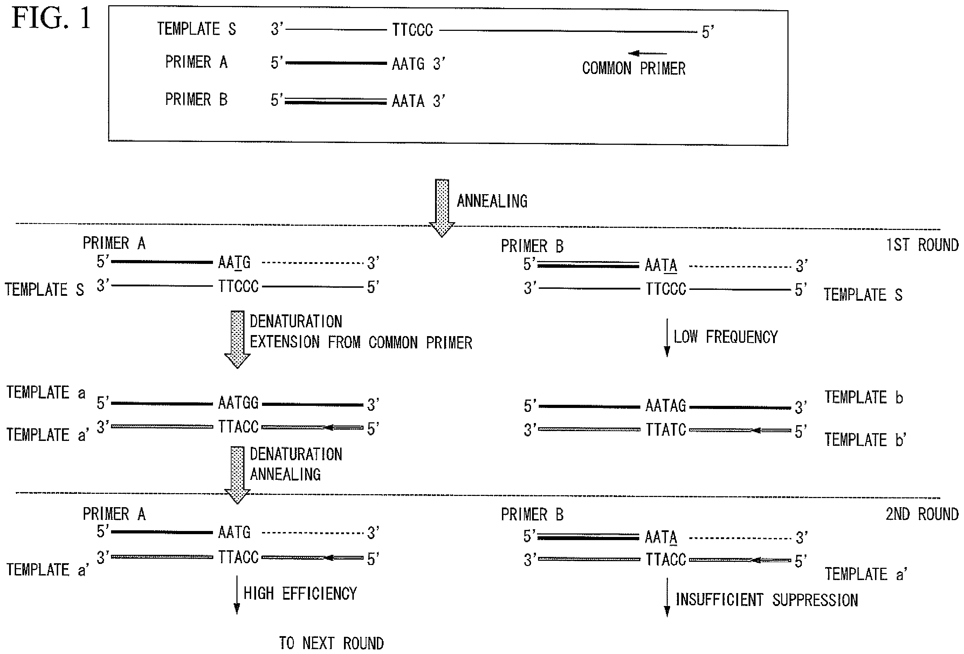

Next is a more detailed description of the method of detecting a target base sequence of the present invention with reference to FIG. 1 to FIG. 6.

FIG. 1 is a schematic diagram showing one aspect of a conventional method of detecting a target base sequence.

FIG. 1 shows, as a part of the reaction composition, a template S having a gene polymorphism (C>T), a detection primer (primer A: corresponding to a forward primer), a competitive primer (primer B: corresponding to a forward primer), and a common primer (corresponding to a reverse primer).

The mismatch sites of the detection primer and the competitive primer which anneal to the template S are underlined.

The primer A is the detection primer. In the primer A, guanine is introduced in the 3' end as a base for detecting the polymorphic base, and thymine is introduced in the second base from the 3' end as a mismatch base that is not complementary to a base other than the polymorphic base.

In addition, the primer B is the competitive primer. In the primer B, adenine is introduced in the 3' end as a mismatch base that is not complementary to the polymorphic base, and thymine is introduced in the second base from the 3' end as a mismatch base that is not complementary to a base other than the polymorphic base.

Here, the base types and the positions of the mutations introduced in the primers A and the B are the same.

Genes are amplified between the common primer and either the detection primer or the competitive primer by simultaneously treating the template S with two types of these primers (primer A and primer B), together with four types of deoxynucleotide triphosphates (dNTPs), DNA polymerase, and the common primer.

Here, in the description of this application, "Round" refers to two cycles in PCR. For example, one Round means a step in which either the detection primer or the competitive primer anneals to a template and then undergoes an extension reaction, and after denaturation, the common primer anneals to the extension product made from the detection primer or the competitive primer and then undergoes an extension reaction from the common primer by using the extension product made from the detection primer or the competitive primer as a template.

In FIG. 1, in the 1 st Round, the double strand formed by the primer A with the template S is more stable than that of the primer B, and therefore the efficiency of the extension reaction from the primer A is higher. The reason is that it is possible to make a big difference between the specific extension efficiency from the primer A and the nonspecific extension efficiency from the primer B, by introducing thymine, which is a mismatch against the template S, in the second bases from the 3' ends of both types of primers.

However, although the frequency is low, the extension product generated from the nonspecific extension reaction serves as a template (template b) for the common primer and is replicated in the next cycle, by which DNA (template b') including the sequence that is complementary to the sequence of the primer B is synthesized. The template a' and the template b' are completely complementary to the primer A and the primer B respectively, and both are extended from the next Round with good efficiency.

Furthermore, in the next Round, the mismatch base of the primer B that is not complementary to a base other than the polymorphic base can match to the template a'. For this reason, the primer B forms a double strand with the template a', and undergoes an extension reaction. Therefore, after the 2nd Round, the effect of the mismatch bases which have been introduced for improving the specificities of the primer A and the primer B will be lost. The number of the template a' is increased in an exponential manner along with the repetition of each Round. Thus, the number of the templates b and b' will be increased along with the increase of the templates a and a', although the efficiency is lower as compared to the specific extension reaction.

Accordingly, this method has room for improvement in the point of how to make a difference in the extension efficiency between the primer A and the primer B with respect to the template.

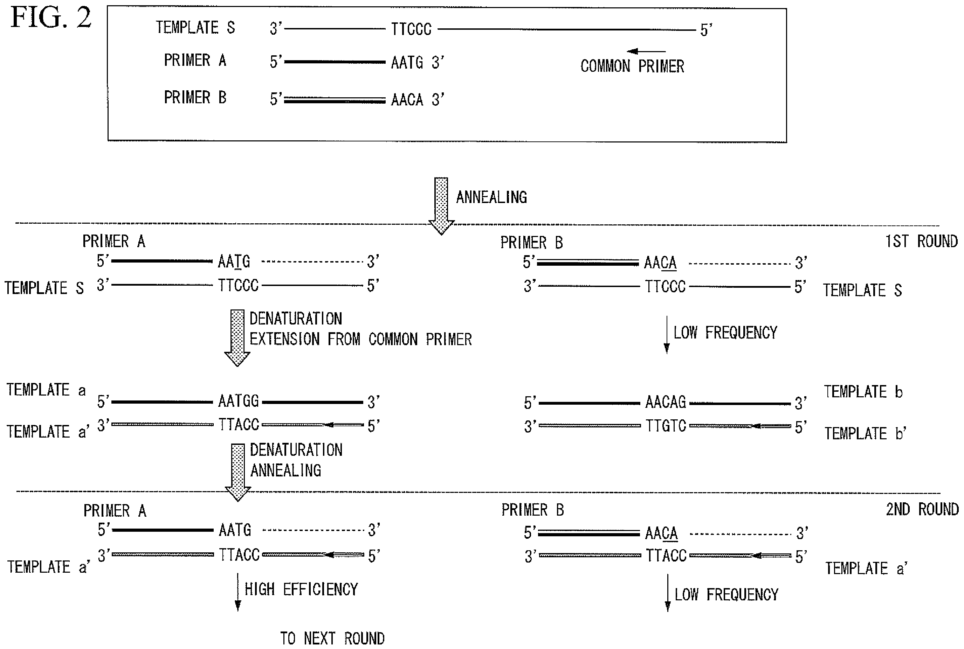

Here is a more detailed description of the conventional method of detecting a target base sequence, with reference to FIG. 2, where different mutations are introduced in the same position between the detection primer and the competitive primer.

FIG. 2 is a schematic diagram showing another aspect of the conventional method of detecting a target base sequence. In the primer A, guanine is introduced in the 3' end as a base for detecting the polymorphic base, and thymine is introduced in the second base from the 3' end as a mismatch base that is not complementary to a base other than the polymorphic base. In addition, in the primer B, adenine is introduced in the 3' end as a mismatch base that is not complementary to the polymorphic base, and cytosine is introduced in the second base from the 3' end as a mismatch base that is not complementary to a base other than the polymorphic base.

Here, the positions of the mutations introduced in the primers A and the B are the same, however the base types are different. As compared to FIG. 1, two bases in the 3' end are mismatched in the nonspecific extension reaction with the template a'. The effect of the mismatch base having been introduced in a position other than the position for detecting the polymorphic base can be sustained, and thus the nonspecific reaction can be suppressed.

However, the number of the template a' keeps increasing in the course of amplification. Therefore, it is necessary to suppress the nonspecific extension reaction more effectively.

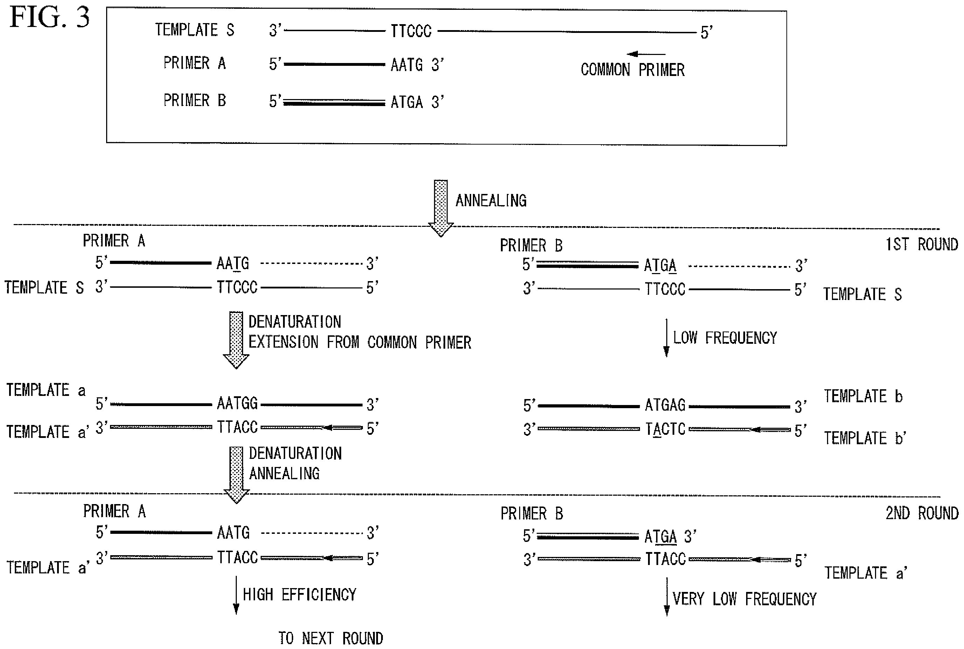

Next is a description of the detection method of the present invention, with reference to FIGs. 3 to 5.

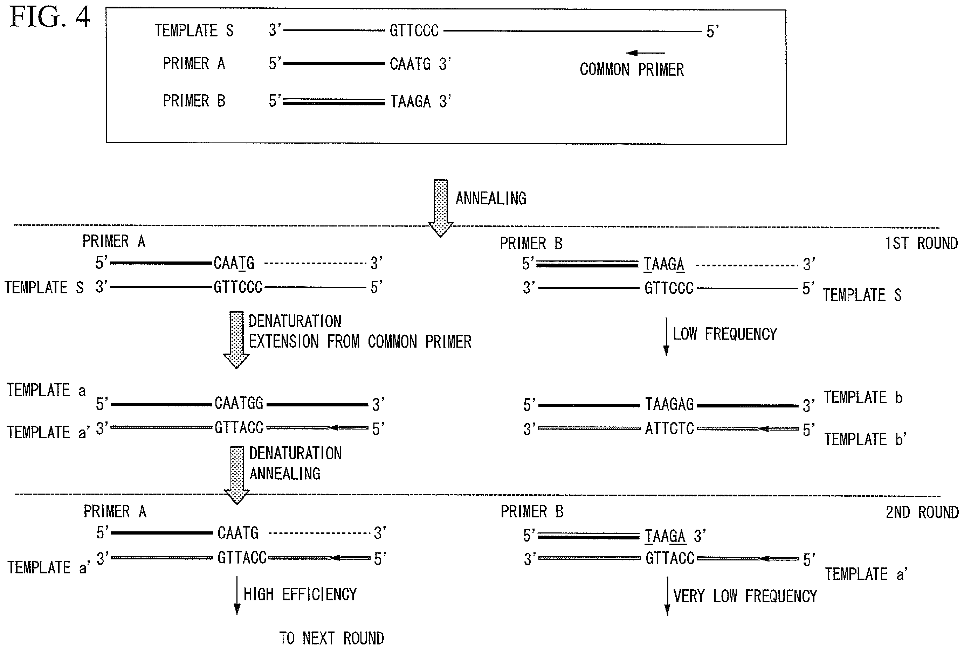

FIG. 3 and FIG. 4 show cases where the position(s) to introduce mismatch base(s) other than the base for detecting the polymorphism is(are) different between the detection primer and the competitive primer in the present invention. The description except for the structures of the primers is omitted as it is the same as the description of FIG. 1. The mismatch sites of the detection primer and the competitive primer which anneal to the template S or the template a' are underlined.

In FIG. 3, the primer A can match to the template S at the 3' end, but is mismatched against the template S at the second base from the 3' end. On the other hand, the primer B is mismatched against the template S at the 3' end and the third base from the 3' end. In this case, after the 2nd Round, three bases in the 3' end of the primer B are mismatched against the template a'. Thus, the nonspecific extension reaction can be quite effectively suppressed. Although the number of the introduced mismatch base other than the base for detecting the polymorphic base is only one, a quite large effect is produced by making a difference in the position between the competitive primers.

In FIG. 4, the primer A can match to the template S at the 3' end, but is mismatched against the template S at the second base from the 3' end. On the other hand, the primer B is mismatched against the template S at the 3' end and the fifth base from the 3' end. Even in such a case, two bases in the 3' end of the primer B are mismatched against the template a' and one base at the fifth base from the 3' end of the primer B is mismatched against the template a'. Thus, the nonspecific extension reaction can be effectively suppressed.

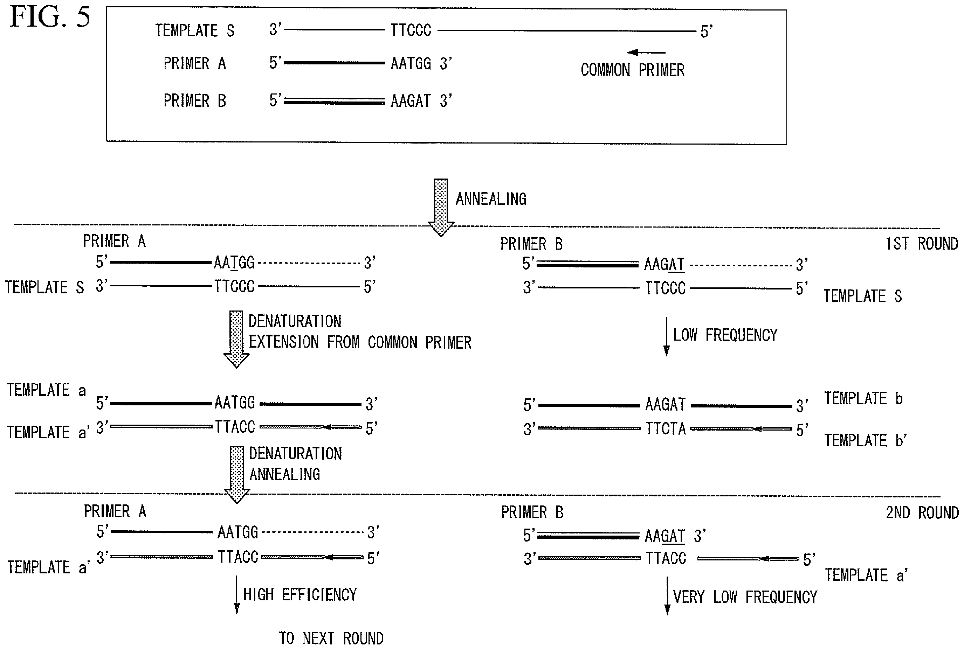

FIG. 5 shows a case where the base for detecting the polymorphic base is located at the second base from the 3' end of the primer in the present invention. Also in this case, although the number of the introduced mismatch base other than the base for detecting the polymorphic base is only one in each primer, three bases in the 3' end of the primer B are mismatched against the template a'. Thus, the nonspecific reaction can be effectively suppressed.

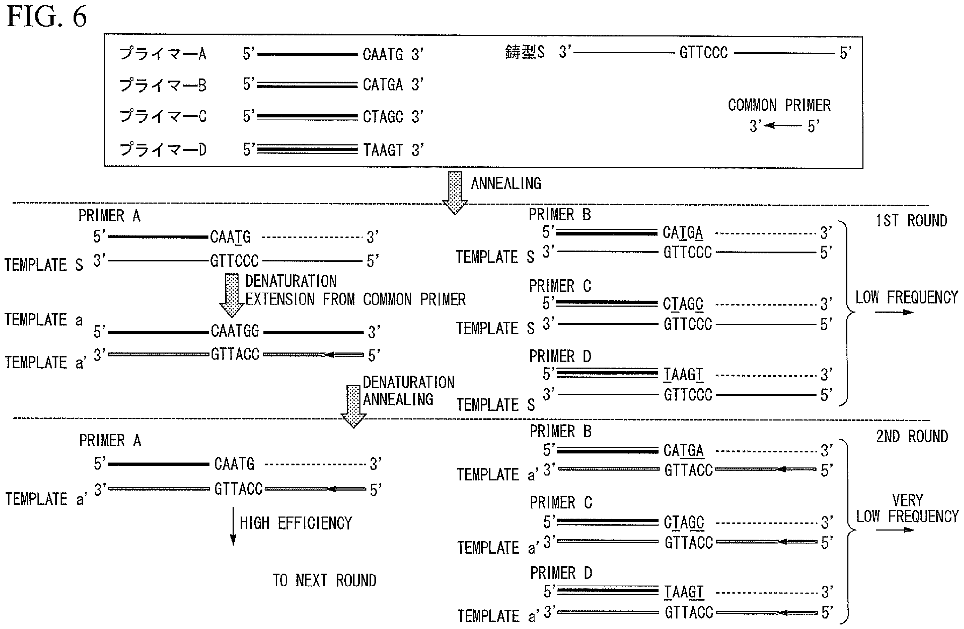

FIG. 6 shows a case where three types of competitive primer are used in the present invention. The description except for the structures of the primers is omitted as it is the same as the description of FIG. 1. The mismatch sites of the detection primer and the competitive primer which anneal to the template S or the template a' are underlined.

In a typical kind of SNP, the number of allele types is two in many cases, but the number may be three in other cases. Furthermore, in the *K-ras* cancer gene, all the possible mutations have been found out in one site. In such a case, it is preferable to have four types of primers respectively corresponding to the four types of bases in a competitive manner.

In FIG. 6, the position to detect each *K-ras* mutation is located at the 3' end of the primer.

The primer A is for detecting the cytosine base. The primer A can match to the template S at the 3' end, and has thymine introduced in the second base from the 3' end as a mismatch base against the template S.

The primer B is for detecting the thymine base. The primer B has a mismatch against the template S at the 3' end, and also has thymine introduced in the third base from the 3' end as a mismatch base against the template S.

The primer C is for detecting the guanine base. The primer C has a mismatch against the template S at the 3' end, and also has thymine introduced in the fourth base from the 3' end as a mismatch base against the template S.

In the same manner, the primer D is for detecting the adenine base. The primer D has a mismatch against the template S at the 3' end, and also has thymine introduced in the fifth base from the 3' end as a mismatch base against the template S.

In this case, the primers B, C, and D respectively have three base mismatches against the template a', and thus the nonspecific extension reaction from each primer can be effectively suppressed.

Accordingly, in the present invention, even if two or more types of competitive allele-specific primers are allowed to compete with each other, it is possible in a practical level to suppress the nonspecific extension reactions by making a difference in the position to introduce a mutation other than the base to identify each mutation, between respective primers.

Simulation was carried out regarding the effect of the present invention as illustrated in FIG. 3 by calculation using Microsoft Excel. Firstly, assuming that the SNP to be detected is cytosine or thymine, four types of primers were designed. The sequences of the templates and the primers are shown in Table 1.

**[Table 1]**

| | SEQUENCE |
|---|---|
| TEMPLATE (C ALLELE) | 3' - · · TTCCC · · - 5' |
| TEMPLATE (T ALLELE) | 3' - · · TTCTC · · - 5' |
| PRIMER [1] FOR DETECTING C ALLELE | 5' - · · AATG - 3' |
| PRIMER [2] FOR DETECTING T ALLELE | 5' - · · AATA - 3' |
| PRIMER [3] FOR DETECTING T ALLELE | 5' - · · AACA - 3' |
| PRIMER [4] FOR DETECTING T ALLELE | 5' - · · ATGA - 3' |

Bold: Position corresponding to the polymorphic base
Underlined: Mismatch base against Template (C allele)

The primer [1] is for detecting the C allele. The primer [1] has guanine at the 3' end, and thymine at the second base from the 3' end as a mismatch against the template.

The primer [2] is for detecting the T allele. The primer [2] has adenine at the 3' end, and thymine at the second base from the 3' end as a mismatch against the template, as with the primer [1].

The primer [3] is for detecting the T allele, as with the primer [2]. However, the primer [3] has cytosine introduced in the second base from the 3' end as a mismatch, which is the difference from the primer [1].

The primer [4] is for detecting the T allele. The primer [4] has thymine introduced in the third base from the 3' end as a mismatch base so that the position of the mismatch base would be different from that of the mismatch base introduced in the primer [1].

The following settings were taken in the simulation.
The initial concentration of the template (template S) was set to be 1.
The rate at which the template S or the extension product (template a' or template b') can form a double strand with each primer was set to be equivalent.
The extension efficiency from the common primer was set to be 1.

Table 2 shows the estimation of the primer extension efficiencies in cases where the primer [1] and any one of the primer [2], the primer [3], or the primer [4] have been competitively reacted. Although the estimation of the extension efficiencies is quite rough, the estimation of the respective number of mismatches and the ranking in the extension reaction efficiency is reasonable. This is based on the assumption in which the template S is the C allele. The symbols [1], [2], [3], and [4] of the double strand respectively correspond to the primer [1], the primer [2], the primer [3], and the primer [4]. The symbol S denotes the template S. The symbol [1]' means an extension product from the common primer with use of the extension product from the primer [1] as a template, the symbol [2]' means an extension product from the common primer with use of the extension product from the primer [2] as a template, and the symbols [3]' and [4]' means the same. The upper row of the sequence shows the sequence of the primer, and the lower row shows the sequence of the template.

Note that, mismatch base(s) against the template is(are) underlined in each primer.

**[Table 2]**

| NAME OF DOUBLE STRAND | SEQUENCE (5' T0 3') | EXTENSION EFFICIENCY |
|---|---|---|
| [1]-S | | 0,8 |
| [2]-S | | 0.01 |
| [1]-[2]' | | 0.5 |
| [2]-[1]' | | 0.5 |
| [1]-[1]' | | 0,9 |
| [2]-[2]' | | 0.9 |
| [3]-S | | 0.01 |
| [1]-[3]' | | 0.01 |
| [3]-[1]' | | 0.01 |
| [3]-[3]' | | 0.9 |
| [4]-S | | 0.01 |
| [1]-[4]' | | 0.001 |
| [4]-[1]' | | 0.001 |
| [4]-[4]' | | 0.9 |

Table 3 to Table 8 show the calculation results up to the 20th Round in the cases where the primer [1] and any one of the primer [2], the primer [3], or the primer [4] have been competitively reacted. Note that, mismatch base(s) against the template is(are) denoted by underlining in each primer.

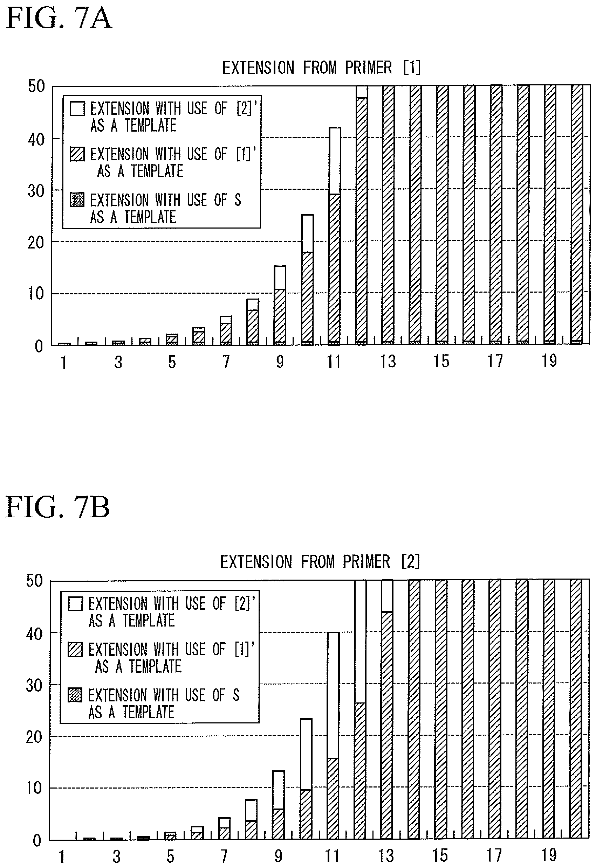

In addition, graphs showing the amount of products extended from the respective primer per each Round are shown in FIGs. 7A to 9B. FIG. 7A shows the breakdown of the templates of the extension products extended from the primer [1], FIG. 7B shows the breakdown of the templates of the extension products extended from the primer [2].

**[Table 3]**

| | EXTENSION PRODUCT FROM PRIMER [1] | | | | | EXTENSION PRODUCT FROM PRIMER [2] | | | | |
|---|---|---|---|---|---|---|---|---|---|---|
| | | | ABUNDANCE OF TEMPLATE | EXTENSION EFFICIENCY | AMOUNT OF AMPLIFICATION | | | ABUNDANCE OF TEMPLATE | EXTENSION EFFICIENCY | AMOUNT OF AMPLIFICATION |
| 1ST ROLIND | [1]-S | | | | | [2]-S | | | | |
| | | | 0.5 | 0.8 | 0.4 | | | 0.5 | 0.01 | 0.005 |
| SUM | | | | | 0.4 | | | | | 0.005 |
| 2ND ROUND | [1]-S | | | | | [2]-S | | | | |
| | | | 0.5 | 0.8 | 0.4 | | | 0.5 | 0.01 | 0.005 |
| | [1]-[1]' | | | | | [2]-[1]' | | | | |
| | | | 0.2 | 0.9 | 0.18 | | | 0.2 | 0.5 | 0.1 |
| | [1]-[2]' | | | | | [2]-[2]' | | | | |
| | | | 0.0025 | 0.5 | 0.00125 | | | 0.0025 | 0.9 | 0.00225 |
| SUM | | | | | 0.58125 | | | | | 0.10725 |
| TOTAL | | | | | 0.98125 | | | | | 0.11225 |
| 3RD ROUND | [1]-S | | | | | [2]-S | | | | |
| | | | 0.5 | 0.8 | 0.4 | | | 0.5 | 0.01 | 0.005 |
| | [1]-[1]' | | | | | [2]-[1]' | | | | |
| | | | 0.490625 | 0.9 | 0.441563 | | | 0.490625 | 0.5 | 0.245313 |
| | [1]-[2]' | | | | | [2]-[2]' | | | | |
| | | | 0.056125 | 0.5 | 0.028063 | | | 0.056125 | 0.9 | 0.050513 |
| SUM | | | | | 0.869625 | | | | | 0.300825 |
| TOTAL | | | | | 1.850875 | | | | | 0.413075 |
| 4TH ROUND | [1]-S | | | | | [2]-S | | | | |
| | | | 0.5 | 0.8 | 0.4 | | | 0.5 | 0.01 | 0.005 |
| | [1]-[1]' | | | | | [2]-[1]' | | | | |
| | | | 0.9254375 | 0.9 | 0.832894 | | | 0.9254375 | 0.5 | 0.462719 |
| | [1]-[2]' | | | | | [2]-[2]' | | | | |
| | | | 0.2065375 | 0.5 | 0.103269 | | | 0.2065375 | 0.9 | 0.185884 |
| SUM | | | | | 1.336163 | | | | | 0.653603 |
| TOTAL | | | | | 3.187038 | | | | | 1.066678 |

**[Table 4]**

| | EXTENSION PRODUCT FROM PRIMER [1] | | | | | EXTENSION PRODUCT FROM PRIMER [2] | | | | |
|---|---|---|---|---|---|---|---|---|---|---|
| | | | ABUNDANCE OF TEMPLATE | EXTENSION EFFICIENCY | AMOUNT OF AMPLIFICATION | | | ABUNDANCE OF TEMPLATE | EXTENSION EFFICIENCY | AMOUNT AMPLIFICATION |
| 5TH ROUND | [1]-S | | | | | [2]-S | | | | |
| | | | 0.5 | 0.8 | 0.4 | | | 0.5 | 0.01 | 0.005 |
| | [1]-[1]' | | | | | [2]-[1]' | | | | |
| | | | 1.5935188 | 0.9 | 1.434167 | | | 1.5935188 | 0.5 | 0.796759 |
| | [1]-[2]' | | | | | [2]-[2]' | | | | |
| | | | 0.5333388 | 0.5 | 0.266669 | | | 0.5333388 | 0.9 | 0.480005 |
| SUM | | | | | 2.100836 | | | | | 1.281764 |
| TOTAL | | | | | 5.287874 | | | | | 2.348442 |
| 10TH ROUND | [1]-S | | | | | [2]-S | | | | |
| | | | 0.5 | 0.8 | 0.4 | | | 0.5 | 0.01 | 0.005 |
| | [1]-[1]' | | | | | [2]-[1]' | | | | |
| | | | 19.062138 | 0.9 | 17.15592 | | | 19.062138 | 0.5 | 9.531089 |
| | [1]-[2]' | | | | | [2]'-[2]' | | | | |
| | | | 14.954355 | 0.5 | 7.477177 | | | 14.954355 | 0.9 | 13.45892 |
| SUM | | | | | 25.0311 | | | | | 22.99499 |
| TOTAL | | | | | 63.15738 | | | | | 52.9037 |
| 15TH ROUND | [1]-S' | | | | | [2]-S | | | | |
| | | | 0.5 | 0.8 | 0.4 | | | 0.5 | 0.01 | 0.005 |
| | [1]-1]' | | | | | [2]-[1]' | | | | |
| | | | 249.24745 | 0.9 | 224.3227 | | | 249.24745 | 0.5 | 124.6237 |
| | [1]-[2]' | | | | | [2]-[2]' | | | | |
| | | | 237.55626 | 0.5 | 118.7781 | | | 237.55526 | 0.9 | 213.8006 |
| SUM | | | | | 343.5008 | | | | | 338.4294 |
| TOTAL | | | | | 841.9957 | | | | | 813.5419 |
| 20T HEROUND | [1]-S | | | | | [2]-S | | | | |
| | | | 0.5 | 0.8 | 0.4 | | | 0,5 | 0,01 | 0.005 |
| | [1]-[1]' | | | | | [2]-[1]' | | | | |
| | | | 3473.1479 | 0.9 | 3125.833 | | | 3473.1479 | 0.5 | 1736.574 |
| | [1]-[2]' | | | | | [2]-[2]' | | | | |
| | | | 3442.5868 | 0.5 | 1721.293 | | | 3442.5868 | 0.9 | 3098.328 |
| SUM | | | | | 4847.527 | | | | | 4834.907 |
| TOTAL | | | | | 11793.82 | | | | | 11720.08 |

**[Table 5]**

| | EXTENSION PRODUCT FROM PRIMES [1] | | | | | EXTENSION PRODUCT FROM PRIMER [2] | | | | |
|---|---|---|---|---|---|---|---|---|---|---|
| | | | ABUNDANCE OF TEMPLATE | EXTENSION EFFICIENCY | AMOUNT OF AMPLIFICATION | | | ABUNDANCE OF TEMPLATE | EXTENSION EFFICIENCY | AMOUNT OF AMPLIFICATION |
| 1ST ROUND | [1]-S | | | | | [3]-S | | | | |
| | | | 0.5 | 0.8 | 0.4 | | | 0.5 | 0.01 | 0.005 |
| SUM | | | | | | | | | | 0.005 |
| 2ND ROUND | [1]-S | | | | | [3]-S | | | | |
| | | | 0.5 | 0.8 | 0.4 | | | 0.5 | 0.01 | 0.005 |
| | [1]-[1]' | | | | | [3]-[1]' | | | | |
| | | | 0.2 | 0.8 | 0.18 | | | 0.2 | 0.01 | 0.002 |
| | [1]-[3]' | | | | | [3]-[3]' | | | | |
| | | | 0.0025 | 0.01 | 0.000025 | | | 0.0025 | 0.9 | 0.00225 |
| SUM | | | | | 0.580025 | | | | | 0.00925 |
| TOTAL | | | | | 0.980025 | | | | | 0.01425 |
| 3RD ROUND | [1]-S | | | | | [3]-S | | | | |
| | | | 0.5 | 0.8 | 0.4 | | | 0.5 | 0.01 | 0.005 |
| | [1]-[1]' | | | | | [3]-[1]' | | | | |
| | | | 0.4800125 | 0.9 | 0.441011 | | | 0.4900125 | 0.01 | 0.0049 |
| | [1]-[3]' | | | | | [3]-[3]' | | | | |
| | | | 0.007125 | 0.01 | 7.13E-05 | | | 0.007125 | 0.9 | 0.006413 |
| SUM | | | | | 0.841083 | | | | | 0.016313 |
| TOTAL | | | | | 1.821108 | | | | | 0.030563 |
| 4TH ROUND | [1]-S | | | | | [3]-S | | | | |
| | | | 0.5 | 0.8 | 0.4 | | | 0.5 | 0.01 | 0.005 |
| | [1]-[1]' | | | | | [3]-[1]' | | | | |
| | | | 0.9105538 | 0.9 | 0.819498 | | | 0.9105538 | 0.01 | 0.009106 |
| | [1]-[3]' | | | | | [3]-[3]' | | | | |
| | | | 0.0152813 | 0.01 | 0.000153 | | | 0.0152813 | 0.9 | 0.013753 |
| SUM | | | | | 1.219651 | | | | | 0.027859 |
| TOTAL | | | | | 3.040759 | | | | | 0.058421 |

**[Table 6]**

| | EXTENSION PRODUCT FROM PRIMER [1] | | | | | EXTENSION PRODUCT FROM PRIMER [2] | | | | |
|---|---|---|---|---|---|---|---|---|---|---|
| | | | ABUNDANCE OF TEMPLATE | EXTENSION EFFICIENCY | AMOUNT OF AMPLIFICATION | | | ABUNDANCE OF TEMPLATE | EXTENSION EFFICIENCY | AMOUNT OF AMPLIFICATION |
| 5TH ROUND | [1]-S | | | | | [3]-S | | | | |
| | | | 0.5 | 0.8 | 0.4 | | | 0,5 | 0.01 | 0.005 |
| | [1]-[1]' | | | | | [3]-[1]' | | | | |
| | | | 1.5203793 | 0.9 | | | | 1.5203793 | 0.01 | 0.015204 |
| | [1]-[3]' | | | | | [3]-[3]' | | | | |
| | | | 0.0292107 | 0.01 | 0.000292 | | | 0.0292107 | 0.9 | 0.02629 |
| SUM | | | | | 1.768634 | | | | | 0.046493 |
| TOTAL | | | | | 4.809392 | | | | | 0.104915 |
| 10TH ROUND | [1]-S | | | | | [3]-S | | | | |
| | | | 0.5 | 0.8 | 0.4 | | | 0.5 | 0.01 | 0.005 |
| | [1]-[1]' | | | | | [3]-[1]' | | | | |
| | | | 12.15431 | 0.9 | 10.93888 | | | 12.15431 | 0.01 | 0.121543 |
| | [1]-[3]' | | | | | [3]-[3]' | | | | |
| | | | 0.4077374 | 0.01 | 0.004077 | | | 0.4077374 | 0.9 | 0.366964 |
| SUM | | | | | 11.34296 | | | | | 0.493507 |
| TOTAL | | | | | 35.65158 | | | | | 1.308982 |
| 15TH ROUND | [1]-S | | | | | [3]-S | | | | |
| | | | 0.5 | 0.8 | 0.4 | | | 0.5 | 0.01 | 0.005 |
| | [1]-[1]' | | | | | [3]-[1]' | | | | |
| | | | 80.364918 | 0.9 | 72.32843 | | | 80.364918 | 0.01 | 0.803649 |
| | [1]-[3]' | | | | | [3]-[3]' | | | | |
| | | | 4.0094937 | 0.01 | 0.040095 | | | 4.0094937 | 0.9 | 3.608544 |
| SUM | | | | | 72.76852 | | | | | 4.417193 |
| TOTAL | | | | | 233.4984 | | | | | 12-43618 |
| 20TH ROUND | [1]-S | | | | | [3]-S | | | | |
| | | | 0.5 | 0.8 | 0.4 | | | 0.5 | 0.01 | 0.005 |
| | [1]-[1]' | | | | | [3]-[1]' | | | | |
| | | | 518.02681 | 0.9 | 466.2241 | | | 518.02681 | 0.01 | 5.180268 |
| | [1]-[3]' | | | | | [3]-[3]' | | | | |
| | | | 34.636853 | 0.01 | 0.346369 | | | 34.6386853 | 0.9 | 31.17317 |
| SUM | | | | | 466.8705 | | | | | 36.35844 |
| TOTAL | | | | | 1503.024 | | | | | 105.6321 |

**[Table 7]**

| | EXTENSION PRODUCT FROM PRIMER [1] | | | | | EXTENSION PRODUCT FROM PRIMER [2] | | | | |
|---|---|---|---|---|---|---|---|---|---|---|
| | | | ABUNDANCE OF TEMPLATE | EXTENSION EFFICI ENCY | AMOUNT OF AMPLIFICATION | | | ABUNDANCE OF TEMPLATE | EXTENSION EFFICIENCY | AMOUNT OF AMPLIFICATION |
| 1ST ROUND | [1]-S | | | | | [4]-S | | | | |
| | | | 0.5 | 0.8 | 0.4 | | | 0.5 | 0.01 | 0.005 |
| SUM | | | | | 0.4 | | | | | 0.005 |
| 2ND ROUND | [1]-S | | | | | [4]-S | | | | |
| | | | 0.5 | 0.8 | 0.4 | | | 0.5 | 0.01 | 0.005 |
| | [1]-[1]' | | | | | [4]-[1]' | | | | |
| | | | 0.2 | 0.9 | 0.18 | | | 0.2 | 0.001 | 0.0002_{.} |
| | [1]-[1]' | | | | | [4]-[4]' | | | | |
| | | | 0.0025 | 0.001 | 2.5E-06 | | | 0.0025 | 0.8 | 0.00225 |
| SUM | | | | | 0.580003 | | | | | 0.00745 |
| TOTAL | | | | | 0.930003 | | | | | 0.01245 |
| 3RD ROUND | [1]-S | | | | | [4]-S | | | | |
| | | | 0.5 | 0.8 | 0.4 | | | 0.5 | 0.01 | 0.005 |
| | [1]-[1]' | | | | | [4]-[1]' | | | | |
| | | | 0.4900013 | 0.9 | 0.441001 | | | | 0.001 | 0.00049 |
| | [1]-[4]' | | | | | [4]-[4]' | | | | |
| | | | 0.006225 | 0.001 | 6.23E-06 | | | 0.006225 | 0.9 | 0.005603 |
| SUM | | | | | 0.841007 | | | | | 0.011093 |
| TOTAL | | | | | 1.82101 | | | | | 0.023543 |
| 4TH RWND | [1]-S | | | | | [4]-S | | | | |
| | | | 0.5 | 0.8 | 0.4 | | | 0.5 | 0.01 | 0.005 |
| | [1]-[1]' | | | | | [4]-[1]' | | | | |
| | | | 0.9105049 | 0.9 | 0.819454 | | | 0.9105049 | 0.001 | 0.000911 |
| | [1]-[4]' | | | | | [4]-[4]' | | | | |
| | | | 0.0117713 | 0.001 | 1.18E-05 | | | 0.0117713 | 0.9 | 0.010594 |
| SUM | | | | | 1.219466 | | | | | 0.016505 |
| TOTAL | | | | | 3.040476 | | | | | 0.040047 |

**[Table 8]**

| | EXTENSION PRODUCT FROM PRIMER [1] | | | | | EXTENSION PRODUCE FROM PRIMER [2] | | | | |
|---|---|---|---|---|---|---|---|---|---|---|
| | | | ABUNDANCE OF TEMPLATE | EXTENSION EFFICIENCY | AMOUNT OF AMPLIFICATION | | | ABUNDANCE OF TEMPLATE | EXTENSION EFFICIENCY | AMOUNT OF AMPLIFICATION |
| 5TH ROUND | [1]-S | | | | | [4]-S | | | | |
| | | | 0.5 | 0.8 | 0.4 | | | 0.5 | 0.01 | 0.005 |
| | [1]-[1]' | | | | | [4]-[1]' | | | | |
| | | | 1.520238 | 0.9 | 1.368214 | | | 1.520238 | 0.001 | 0.00152 |
| | [1]-[4]' | | | | | [4]-[4.]' | | | | |
| | | | 0.0200236 | 0.001 | 2E-05 | | | 0.0200236 | 0.9 | 0.018021 |
| SUM | | | | | 1.768234 | | | | | 0.024541 |
| TOTAL | | | | | 4.80871 | | | | | 0.064589 |
| 10TH ROUND | [1]-S | | | | | [4]-S | | | | |
| | | | 0.5 | 0.8 | 0.4 | | | 0.5 | 0.01 | 0.005 |
| | [1]-[1]' | | | | | [4]-[1]' | | | | |
| | | | 12.148909 | 0.9 | 10.93402 | | | 12.148909 | 0.001 | 0.012149 |
| | [1]-[4]' | | | | | [4]-[4]' | | | | |
| | | | 0.1774407 | 0.001 | 0.000177 | | | 0.1774407 | 0.9 | 0.159697 |
| SUM | | | | | 11.3342 | | | | | 0.176846 |
| TOTAL | | | | | 35.63201 | | | | | 0.531727 |
| 15TH ROUND | [1]-S | | | | | [4]-S | | | | |
| | | | 0.5 | 0.8 | 04 | | | 0.5 | 0.01 | 0.005 |
| | [1]-[1]' | | | | | [4]-[1]' | | | | |
| | | | 88.277688 | 0.9 | 72.24992 | | | 80.277688 | 0.001 | 0.080278 |
| | [1]-[4]' | | | | | [4]-[4]' | | | | |
| | | | 1.3039044 | 0.001 | 0.001304 | | | 1.3039044 | 0.9 | 1.173514 |
| SUM | | | | | 72.65122 | | | | | 1.258792 |
| TOTAL | | | | | 233.2066 | | | | | 3.8666 |
| 20TH ROUND | [1]-S | | | | | [4]-S | | | | |
| | | | 0.5 | 0.8 | 0.4 | | | 0.5 | 0.01 | 0.005 |
| | [1]-[1]' | | | | | [4]-[1]' | | | | |
| | | | 516.97801 | 0.9 | 465.2802 | | | 516.97801 | 0.001 | 0.516978 |
| | [1]-[4]' | | | | | | | | | |
| | | | 9.27715.5 | 0.001 | 0.009277 | | | 9.277155 | 0.9 | 8.349439 |
| SUM | | | | | 465.6895 | | | | | 8.871417 |
| TOTAL | | | | | 1499.645 | | | | | 27.42673 |

FIGs. 7A and 7B show that, when using the combination of the primers [1] and [2], the extension is carried out with high frequency even if the template and the primer are mismatched. Thus, the amount of the extension product from the primer [2] is large. Particularly, when [1]' and the primer [2] have formed a double strand to undergo an extension reaction (extension with use of [1]' as a template in FIG. 7B). a template that can match to the primer [2] is produced (extension with use of [2]'as a template in FIG. 7B) from the next Round. Therefore, in the result, the amount of the extension product from the primer [2] is increased more. Moreover, [2]' is also able to serve as a template of the primer [1] (extension with use of [2]'as a template in FIG. 7A). Thus, the amount of false positive extension product from the primer [1] is large.

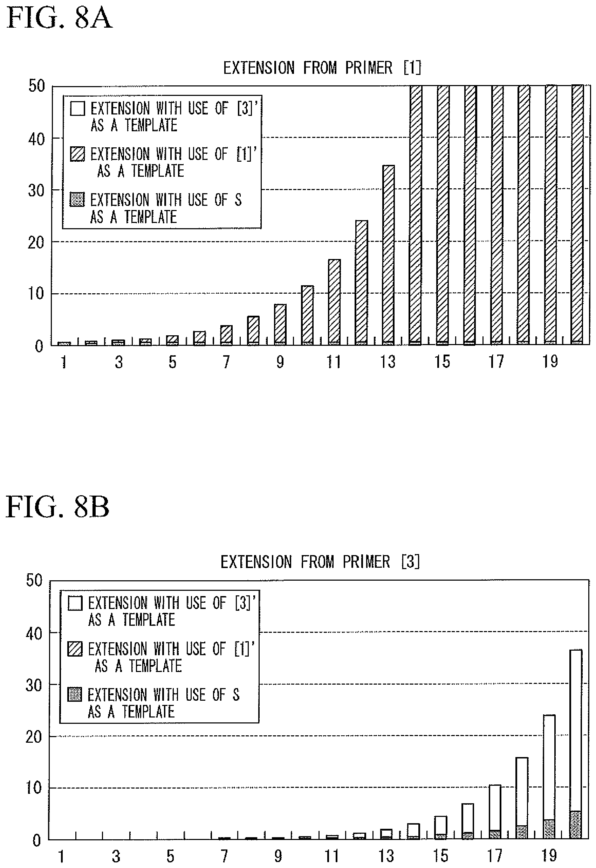

FIGs. 8A and 8B show that, when using the combination of the primers [1] and [3], the extension reaction is suppressed to some degree if the template and the primer are mismatched. Thus, the amount of the extension product from the primer [3] is small. When [1]' and the primer [3] have formed a double strand to undergo an extension reaction (extension with use of [1]' as a template in FIG. 8B), a template that can match to the primer [3] is produced (extension with use of [3]'as a template in FIG. 8B) from the next Round. Therefore, the amount of the extension product from the primer [3] is increased more.

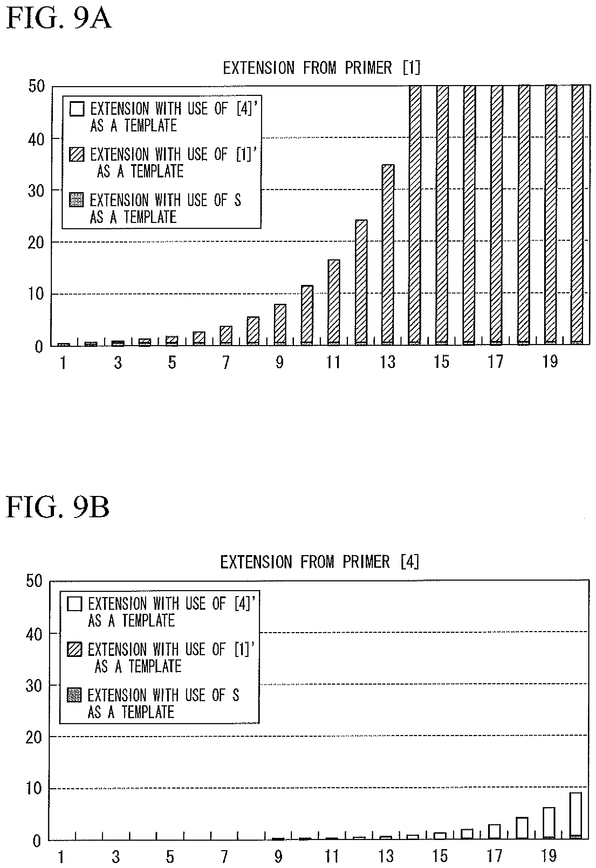

FIGs. 9A and 9B show that, when using the combination of the primers [1] and [4], the extension reaction hardly occurs if the template and the primer are mismatched. Thus, the amount of the extension product from the primer [4] made by using [1]' as a template (extension with use of [1]' as a template in FIG. 9B) is only a few. Accordingly, even if the amount of the extension product from the primer [1] is increased, the amount of the extension product from the primer [4] is not increased so much.

From the simulation results, the identification precision was higher in the combination of the primers [1] and [4] rather than the combination of the primers [1] and [2] and the combination of the primers [1] and [3]. Accordingly, it was confirmed that the improvement of the identification precision was led by making a difference in the position of the mismatch base introduced in both the detection primer and the competitive primer.

The method of detecting a target base sequence of the present invention includes (a) a step of adding to a nucleic acid sample having the target nucleic acid that includes a base sequence including the target base sequence: at least one type of detection primer that is substantially complementary to the target base sequence, at least one type of competitive primer that is substantially complementary to the target base sequence as well as being capable of annealing to a target nucleic acid, in a competitive manner against the detection primer, and at least one type of common primer, (b) a step of annealing the detection primer and the competitive primer to the target nucleic acid in a competitive manner with use of the target base sequence having the polymorphic base in the nucleic acid sample as a template, thereby causing an extension reaction to synthesize an extension product A, (c) a step of annealing the common primer to the extension product A obtained in the step (b) or in the following step (d), thereby causing an extension reaction to synthesize an extension product B, (d) a step of annealing the detection primer or the competitive primer to the extension product B obtained in the step (c), thereby synthesizing the extension product A, and (e) a step of detecting the extension product A or the extension product B.

Hereunder is a description of the respective steps.

Firstly, in the step (a), one type of detection primer, at least one type of competitive primer, and at least one type of common primer are added to a nucleic acid sample having a target nucleic acid that includes a base sequence including the target base sequence.

The nucleic acid sample is not specifically limited as long as it contains a nucleic acid. The sample is preferably prepared by nucleic acid extraction from an animal, a plant, a microbe, cultured cells, or the like. The nucleic acid extraction from an animal or the like can be conducted by a known method such as the phenol/chloroform method. Note that, in the case where the nucleic acid contained in the nucleic acid sample is a double stranded nucleic acid, it is preferable to separate it into single stranded nucleic acids in advance. By using single stranded nucleic acids, it is possible to enable the detection primer and the competitive primer to anneal to these single stranded nucleic acids in the step (b) that will be described later. The separation of the extracted double stranded nucleic acid into single stranded nucleic acids can be performed by a known method such as the application of thermal energy.

The type of the nucleic acid in the nucleic acid sample is not specifically limited as long as it is either DNA or RNA. The nucleic acid may be either a natural substance or a synthesized one. The natural nucleic acid can be exemplified by genome DNA, mRNA, rRNA, hnRNA, or the like, collected from an organism. Moreover, the synthesized nucleic acid can be exemplified by DNA synthesized by a known chemical synthesis method such as the β-cyanoethyl phosphoramidite method, or the DNA solid-phase synthesis method, a nucleic acid synthesized by a known nucleic acid synthesis method such as PCR, cDNA synthesized by reverse transcription reaction, or the like.

Note that, in the present invention, the term "oligonucleotide" refers to a substance having the same function as that of deoxyribonucleotide (DNA) and ribonucleotide (RNA), whether or not it is naturally derived or not. This term also includes artificial nucleic acids such as PNA and LNA.

In the present invention, the term "primer" refers to a short nucleic acid fragment which plays a role to supply 3' hydroxyl group when a DNA polymerase or a reverse transcriptase starts to synthesize DNA after having a double stranded state with a template.

Next, in the step (b), the detection primer and the competitive primer are annealed to the target nucleic acid in a competitive manner with use of the target base sequence having the polymorphic base in the nucleic acid sample, so as to cause an extension reaction to synthesize an extension product A.

The reaction condition where the detection primer or the competitive primer anneals to the target nucleic acid is not specifically limited. The reaction can be performed under usual conditions regarding the temperature, the pH, the salt concentration, the buffer solution, and the like, with consideration of the Tm values of the respective primers, and the like.

In the present invention, the term "extension reaction" refers to a reaction to synthesize a nucleic acid conducted by using reagents such as dNTPs, a DNA polymerase, and the like. This term also includes an extension reaction by means of a reverse transcriptase in which RNA is adopted as a template.

The term "DNA polymerase" is a generic expression referring to an enzyme which helps to synthesize a DNA strand having a base sequence that is complementary to the template DNA annealed by a primer.

The DNA polymerase for use in the present invention is not specifically limited, although it is preferable to use a Taq DNA polymerase, a Tth DNA polymerase, a Vent DNA polymerase, and such a thermostable DNA polymerase. In order to prevent an extension before starting the test, it is more preferable to use a DNA polymerase having a hot start function. Furthermore, in the present invention, in order to identify the base in the vicinity of the 3' end of the primer, it is particularly preferable to use a DNA polymerase not having the 3'-to-5' exonuclease activity.

In addition, regarding specific methods such as for the reaction conditions for performing this extension reaction, it is possible to implement these by referring to known methods described in documents such as Jikken Igaku (Experimental Medicine) Vol. 8, No. 9 (Yodosha Co., Ltd. (1990)), PCR Technology Stockton Press (1989), and the like.

Next, in the step (c), the common primer is annealed to the extension product A obtained in the previous step (b) or in the following step (d), so as to cause an extension reaction to synthesize an extension product B. In the step (d), the detection primer or the competitive primer is annealed to the extension product B obtained in the previous step (c), so as to synthesize the extension product A. The target nucleic acid is amplified through these steps.

The above-mentioned nucleic acid amplification step can be conducted by PCR (Polymerase Chain Reaction), LAMP (Loop-Mediated Isothermal Amplification), NASBA (Nucleic Acid Sequence Based Amplification), ICAN (Isothermal and Chimerical primer-initiated Amplification of Nucleic acids), TRC (Transcription Reverse-Transcription Concerted), SDA (Strand Displacement Amplification), TMA (Transcription Mediated Amplification), SMAP (SMart Amplification Process), RPA (Recombines Polymerase Amplification), HDA (Helicase-Dependent Amplification), or the like.

If the isothermal amplification reaction is employed in the nucleic acid amplification step mentioned above, it follows a usual method.

In the PCR reaction, regarding the concentrations of the detection primer, the competitive primer, or the common primer, it is possible to appropriately examine their optimum concentrations. However it is preferable to set the concentration of the common primer to be equal to or lower than the total concentration of the detection primer and the competitive primer, and furthermore, more preferably 25% or lower than the total concentration of the detection primer and the competitive primer. This is for the purpose of preventing a lowering of the identification precision caused by a situation where, as the PCR proceeds, the detection primer that can match to the template is preferentially consumed, and the relative concentration of the competitive primer that is a mismatch against the template is increased. By lowering the concentration of the common primer, it is possible to maintain the identification precision, because the common primer is also consumed along with the consumption of the detection primer that can match to the template.

Next, in the step (e), the extension product A or B is detected.

The method of detecting the extension product from the primer in the step (e) is not specifically limited. The method can be exemplified by any method capable of analyzing a nucleic acid, such as; labeling the primer with a fluorophore or the like, electrophoresis, high-performance liquid chromatography, mass spectroscopy, analysis of a melting curve, and analysis of a growth curve.

The extension product can be detected by using labeling substances as indexes by having the detection primer, the competitive primer, or the common primer labeled with these labeling substances. Such labeling substances can be exemplified by a fluorophore, an energy absorbing material, a radioisotope, a chemical luminescent material, an enzyme, an antibody, or the like. The position to label it in each primer is not specifically limited, although a position which would not interfere with the extension reaction is preferable.

It is preferable to adopt a method in which these primers are labeled with different types of fluorophores so as to discriminate which type of primer has been used for the amplification, since the method is easy and convenient with no need of measuring the melting curve, and the influence on the specificity can be little.

It is more preferable to adopt a method in which fluorescein and acridine are introduced in the 5' end of a primer (refer to Non-patent Document 9). When this primer is present in a single stranded state; then, even if light having a wavelength for exciting fluorescein is irradiated, fluorescence from fluorescein is not observed because it is quenched by acridine which serves as an energy absorbing material.

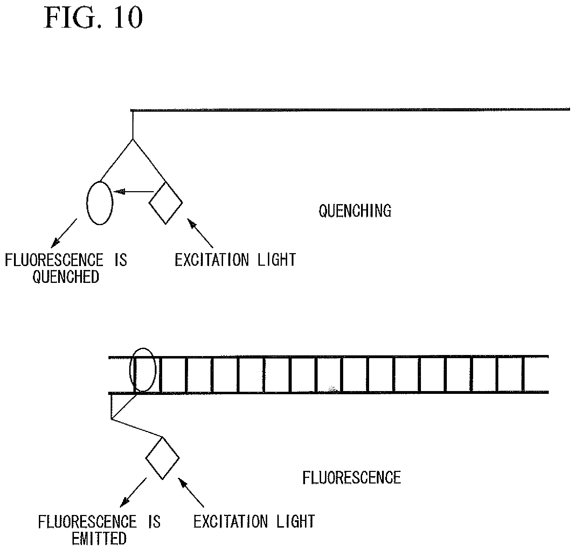

On the other hand, when the primer forms a double strand, the acridine becomes unable to absorb the fluorescence from fluorescein because acridine also serves as an intercalator to bind to the double stranded nucleic acid and therefore the distance between fluorescein and acridine becomes large. Therefore, fluorescence is emitted only from the primer which has undertaken the primer extension reaction (refer to FIG. 10).

Accordingly, it is possible to determine which type of primer, out of the detection primer and the competitive primer, has been used for the extension product by having respectively different types of fluorophores therein.

Therefore, in the detection method of the present invention, it is possible not only to detect the presence or absence of one polymorphism in a sample but also to detect a plurality of polymorphisms in the sample, for example, by: using a primer for detecting the mutant-type allele as a detection primer and a primer for detecting the wild-type allele as a competitive primer, out of two polymorphisms consisting of the wild-type and the mutant type; respectively labeling the detection primer and the competitive primer with different types of fluorophores; and respectively detecting the extension product from each type of primer.

The position to introduce each fluorophore is not specifically limited, although a position which would not interfere with the polymerase reaction is preferable because the competitive primer is labeled.

Moreover, it is also possible to use pyrene instead of acridine. Since pyrene is also energy-absorbable and bindable to a double strand, it is possible to investigate the reaction to form a double strand in the same manner as for acridine.

Besides this, as to the method of detecting which type of primer out of two or more types of allele-specific primers, has been used for the extension, there are some methods that can distinguish the single stranded state and the double stranded state of the primer, such as LUX (trademark) primer (a product manufactured by Invitrogen), Amplifluor (trademark), UNIprimer (trademark) (a product manufactured by CHEMICON) (Ranasinghe et. al., Chem. Commun, Vol. 44, pp. 5487-5502, 2005), any method of which can be applied.

A preferred example of the detection method using a fluorophore can include a detection method using the QP (Quenching Probe/Primer) method.

The QP method is a detection method utilizing the phenomenon in which fluorescence from a certain type of fluorophore is quenched by a guanine base when the guanine base is spatially in the proximity to the fluorophore.

It is possible to detect whether the detection primer and the target nucleic acid are in the proximity or not, by labeling the detection primer of the present invention with such a fluorophore whose fluorescence is quenched by the guanine base located in the proximity thereto. The primer having the guanine base may be either the target nucleic acid or the detection primer, although it is preferably the detection primer.

The above-mentioned fluorophore whose fluorescence is quenched by the guanine base located in the proximity thereto can be a usual fluorophore for use in the QP method. For example, BODIPY FL (product name; manufactured by Invitrogen), PACFIC BLUE (product name; manufactured by Invitrogen), CR6G (product name; manufactured by Invitrogen), TAMRA (product name; manufactured by Invitrogen), and the like, can be enumerated.

When it comes to a detection by means of electrophoresis, it is possible, by making a variation in the lengths of two competitive primers so as to make a difference in the lengths of the extension products thereof, to conduct the detection on the basis of the difference in the mobility. However, it is important not to impose an influence on the specificity by making a variation in the lengths of these primers.

Regarding the reagents for use in the detection by means of electrophoresis, ethidium bromide and SYBR Green are more preferred since they can emit fluorescence by binding to double stranded DNA.

In addition, since SYBR Green is a fluorophore, it is possible to discriminate which type of primer has been used for the extension product, by measuring the melting curve that will be described later.

It is also possible, by means of high-performance liquid chromatography, to discriminate which type of primer out of two primers has been used for the extension product in the same manner.

Moreover, if mass spectrometry is adopted, it is possible to make a difference in the lengths of the extension products from two types of primers, or to label these primers with different substances having different mass weights. The latter case is preferable since the effect onto the specificity is little.

The above-mentioned step (e) may be provided simultaneously with the nucleic acid amplification step including the above-mentioned steps (b) to (d), or may be performed after the nucleic acid amplification step.

The identification method in the nucleic acid amplification step can be exemplified by the method to measure the double stranded state of the labeled primer or the extension product thereof. This identification method utilizes the difference in the capability to form double strands, of the competitive primers which form double strands with the template, or of the extension products from the competitive primers.

Moreover, it is also possible to measure fluorescence under a temperature at which the extension products from the primers can form double strands. Since this method is capable of detecting the product extended from the competitive primer only, more highly precise identification is possible.

The identification method after the nucleic acid amplification step can be exemplified by the method to measure the melting curve. This is a method which utilizes the temperature dependency of the amplification product to transition from the double strand state to the single stranded state. Since this method is capable of detecting the amplification products from these two competitive primers only, more highly precise identification is possible.

The detection method of the present invention can be used for DNA sequencing machines such as a real-time PCR apparatus. In this case, the specimen DNA is placed in a container which contains the above-mentioned reagents required for PCR and the primers, and is subjected to real-time PCR. By so doing, the extension product from the detection primer is detected. Splashing and contamination of the amplification product can be avoided by conducting the detection while sealing the container after placing the specimen DNA.

The target base sequence detection kit of the present invention is a kit for use in the above-mentioned method of detecting a target base sequence having a polymorphic base, wherein the kit includes the detection primer, the competitive primer, and the common primer as mentioned above.

In addition, a cell disruption reagent for use in the pretreatment of the sample, a reagent for detecting the label of the labeling substance, and the like, may also be combined therein.

In this way, by having such reagents and the like required for the method of detecting a target base sequence of the present invention in a kit, single base polymorphism can be identified more easily and conveniently in a shorter time.

Hereunder is a more specific description of the present invention with reference to Examples. However, the present invention is not to be limited by the following Examples.

### [Examples]

In Comparative Examples 1 and 2 and Examples 1 and 2, a gene polymorphism (1173C>T) in the position 1173 of the intron 1 region of the vitamin K epoxide reductase complex 1 (*VKORC1*)*,* which is a gene related to the optimum dose of warfarin, was used as an identification target.

As shown in Table 9, two types of detection primers (VK1Wat-Acridine and VK1Mtg-Acridine) for detecting the *VKORC1* gene polymorphism as mentioned above, and a common primer (VK1R2), were prepared. Regarding the detection primers, those in which acridine had been introduced in the 5' end by using acridine phosphoramidite (Glen Research), and furthermore, 6-fluorescein (Glen Research) had been introduced in the 5' end thereof, were purchased from Japan Bio Services. Regarding four types of competitive primers (VK1Mtg, VK1Mat, VK1Mac, and VK1Wat) and the common primer (VK1R2) shown in Table 9, those synthesized by a usual synthesis method were purchased from Greiner Bio-One Japan. The genome DNA serving as a template was purchased from Coriell.

In Table 9, the position 1173 and the polymorphic base recognition site are shown by bold and the mismatch base introduction site is underlined. In addition, "Acridine" denotes acridine, "6-FAM" denotes the 6-fluorescein label, and the number on the right column denotes the sequence number corresponding to the sequence before labeling as shown in the Sequence Listing.

**[Table 9]**

| TEMPLATE | | |
|---|---|---|
| WILD-TYPE TEMPLATE (C ALLELE) | | 1 |
| MUTANT-TYPE TEMPLATE (T ALLELE) | | 2 |
| NAME OF OLIGONUCLEOTIDE | BASE SEQUENCE | |
| DETECTION PRIMER | | |
| VK1Wat-Acridine | 5'-6-FAM-Acridine-TACCTCCCATCCTAGTCCAATG-3' | 3 |
| VK1Mtg-Acridine | 5'-6-FAM-Acridine-ACCTCCCATCCTAGTCCATGA-3' | 4 |
| COMPETITIVE PRIMER | | |
| VK1Mat | 5'-ACCTCCATCCTAGTCCAATA-3' | 5 |
| Vk1Mec | 5'-ACCTCCCATCCTAGTCCAACA-3' | 6 |
| VK1Mtg | 5'-ACCTCCCATCCTAGTCCATGA-3' | 7 |
| VK1Wat | 5'-ACCTOCCATCCTAGTCCAATG-3' | 8 |
| COMMON PRIMER | | |
| VK1R2 | 5'-AAAAGCAGGGCCTACG-3' | 9 |

### (Comparative Example 1)

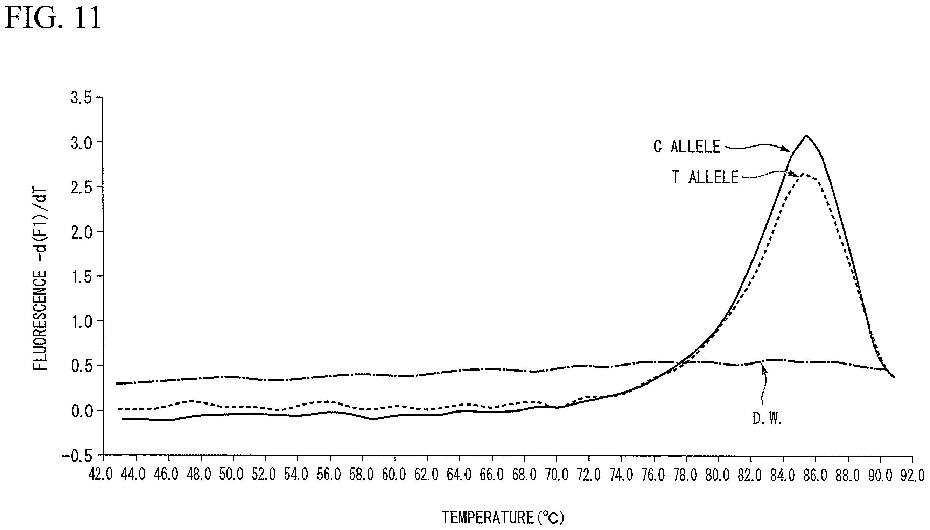

A reaction solution having a mixture of the C allele or the T allele of the *VKORC1* gene polymorphism as a template, VK1Wat-Acridine as a detection primer, VK1Mat as a competitive primer, and VK1R2 as a common primer, was prepared so that the composition would be as of Table 10. This reaction solution was set in the Real-time PCR system (a product manufactured by Roche, "Light Cycler") and held at 95°C for 1 minute, to effect denaturation of the antibody of the DNA polymerase. Then, two-step PCR consisting of 62°C for 20 seconds and 95°C for 5 seconds was run for 55 cycles. The melting curve analysis was carried out from 95°C to 40°C. A solution using distilled water (D.W.) as a template was employed as the negative control. The results are shown in FIG. 11.

**[Table 10]**

| | |
|---|---|
| 5X Color less GoTaq Buffer (Promega) | 2.0 *µ*l |
| 25mM MgCl₂ (Promega) | 1.0 *µ*l |
| 2.5mM each dNTP (Promega) | 0.8 *µ*l |
| 2.5uM DETECTION PRIMER | 1.3 *µ*l |
| 2.5uM COMPETITIVE PRIMER | 1.3 *µ*l |
| 2.5uM COMMON PRIMER | 0.7 *µ*l |
| 5U/ul GoTaq Hot Start Polymerase (Promega) | 0.1 *µ*l |
| 3ng/ul TEMPLATE (Coriell) | 1.0 *µ*l |
| DISTILLED WATER | BALANCE |
| TOTAL VOLUME OF REACTION SOLUTION | 10.0 *µ*l |

FIG. 11 shows the results of Comparative Example 1 expressed by negative first differential curves of the melting curves thereof.

When the detection primer (VK1Wat-Acridine) forms a double strand, acridine serving as the energy absorbing material is intercalated into the double stranded nucleic acid. Thus, the level of energy absorption by acridine is reduced, and thereby the fluorescence intensity from 6-fluorescein increases.

The "melting curve" refers to a curve obtained by measuring the fluorescence intensity until a double stranded nucleic acid amplified by the PCR method is denatured into a single stranded state, through gradient increase of the temperature from low to high. The negative first differential curve of the melting curve represents the changed fluorescence level with respect to the temperature change. The maximum point of the changed level corresponds to the Tm value of the double strand DNA.

In Comparative Example 1, it has been already known that the extension product from VK1Wat-Acridine has a maximum changed level from the double stranded state to the single stranded state within a range from 83°C to 86°C. Accordingly, it is possible to determine that the extension reaction from the VK1Wat-Acridine has occurred if a peak is seen within a range from 83°C to 86°C in the negative first differential curve of the melting curve.

In the case of using a set of the detection primer and the competitive primer of Comparative Example 1, similar results were obtained by using any one of the C allele and the T allele as a template. This suggests that the extension reaction using the above-mentioned primer set is not specific to the C allele.

### (Comparative Example 2)

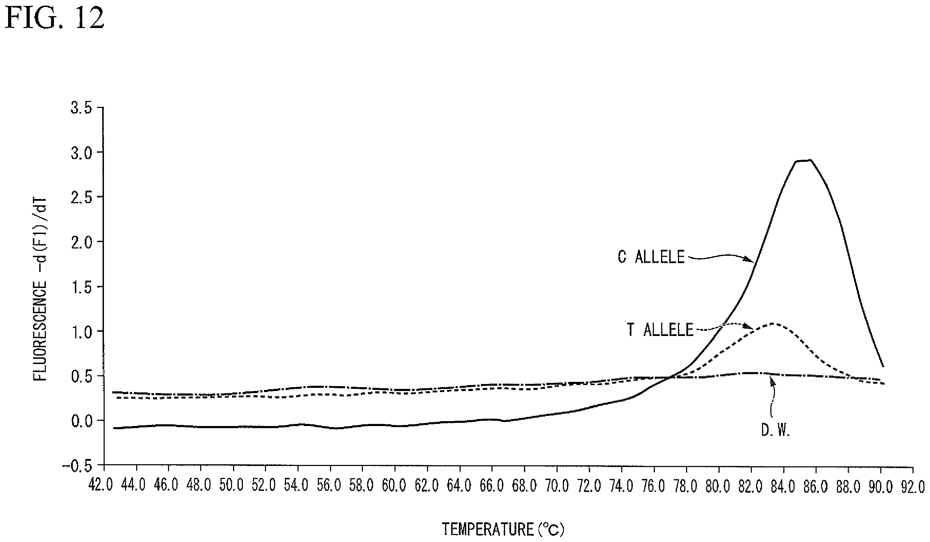

The reaction and the analysis were conducted in the same manner as for Comparative Example 1 except for using VK1Wat-Acridine as a detection primer and VK1Mac as a competitive primer. The results are shown in FIG. 12.

FIG. 12 shows the results of Comparative Example 2 expressed by negative first differential curves of the melting curves thereof.

In the case of using a set of the detection primer and the competitive primer of Comparative Example 2, the amplification from VK1Wat-Acridine was predominantly observed when using the C allele as a template. However, only a small peak was seen when using the T allele as a template. This suggests that the extension from VK1Wat-Acridine was not sufficiently suppressed when using the T allele as a sample.

### (Example 1)

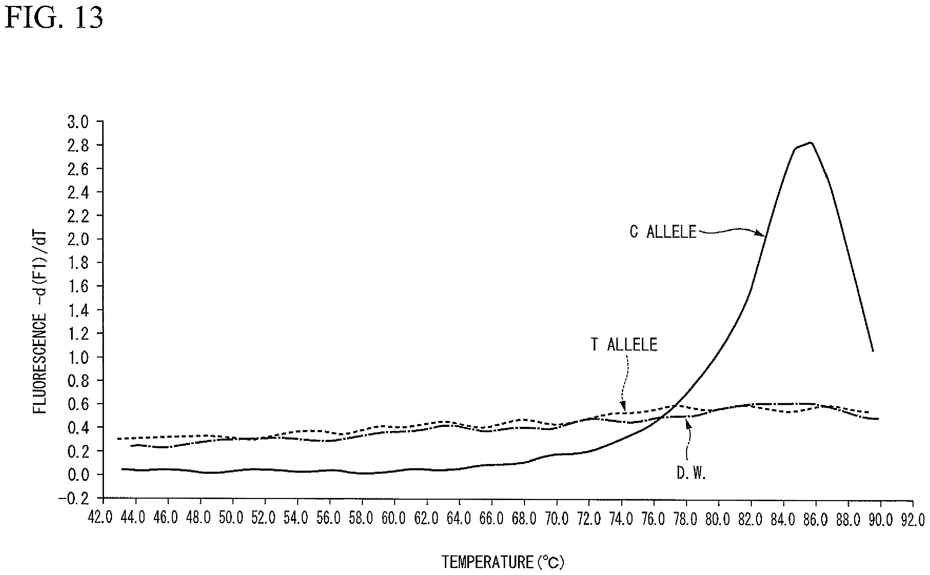

The reaction and the analysis were conducted in the same manner as for Comparative Example 1 except for using VK1Wat-Acridine as a detection primer and VK1Mtg as a competitive primer. The results are shown in FIG. 13.

FIG. 13 shows the results of Example 1 expressed by negative first differential curves of the melting curves thereof.

In the case of using a set of the detection primer and the competitive primer of Example 1, the amplification from VK1Wat-Acridine was predominantly observed when using the C allele as a template. Furthermore, when using the T allele as a template, the amplification from VK1Wat-Acridine was much suppressed as compared to the result of Comparative Example 2. This suggests that the above-mentioned primer set has better C allele specificity.

### (Example 2)

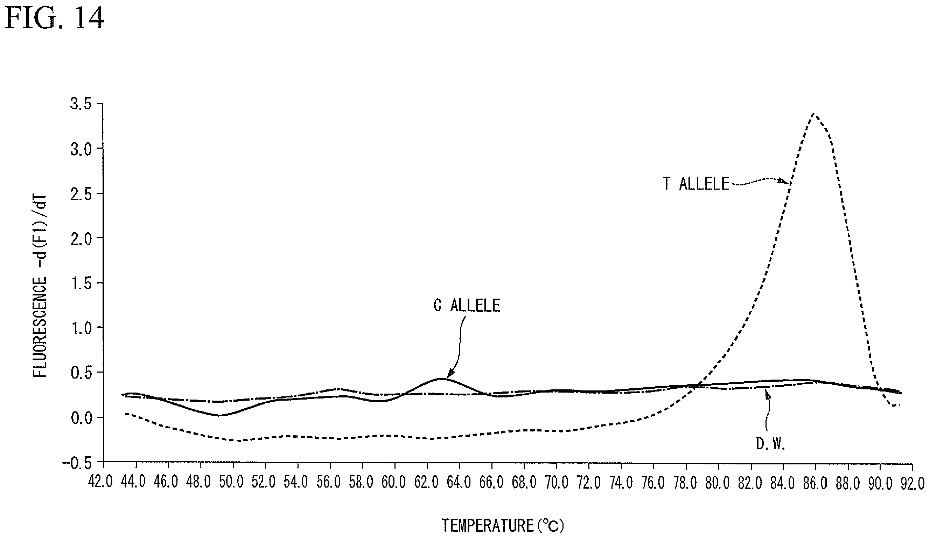

The reaction and the analysis were conducted in the same manner as for Comparative Example 1 except for using VK1Mtg-Acridine as a detection primer and VK1Wat as a competitive primer. The results are shown in FIG. 14.

FIG. 14 shows the results of Example 2 expressed by negative first differential curves of the melting curves thereof.

In Example 2, it has been already known that the extension product from VK1Mtg-Acridine has a maximum changed level from the double stranded state to the single stranded state within a range from 83°C to 86°C. Accordingly, it is possible to determine that the extension reaction from the VK1Mtg-Acridine has occurred if a peak is seen within a range from 83°C to 86°C in the negative first differential curve of the melting curve.

In the case of using a set of the detection primer and the competitive primer of Example 2, the amplification from VK1Mtg-Acridine was predominantly observed when using the T allele as a template. This suggests that the extension reaction using the above-mentioned primer set is not specific to the T allele.

The primer set of Example 2 is the same as the primer set of Example 1 in terms of the set of the base sequences, and the only difference is that the fluorescent label had been introduced in the mutant type primer. Accordingly, from the results of Examples 1 and 2, it was revealed that the presence or absence of the extension reaction from the fluorescence-labeled primer was able to be determined regardless of which one of VK1Wat or VK1Mtg had been labeled with fluorescence.

Accordingly, it is possible to simultaneously measure the extension reactions from the respective fluorescence-labeled primers, by respectively introducing different fluorophores that have mutually different fluorescent wavelengths, into VK1Wat and VK1Mtg.

In Examples 3 to 5, similarly to the above-mentioned cases, a gene polymorphism (1173C>T) in the position 1173 of the intron 1 region of the vitamin K epoxide reductase complex 1 (*VKORC1*), which is a gene related to the optimum dose of warfarin, was used as an identification target, so as to investigate the influence of the position to introduce a mismatch base other than the polymorphic base in the competitive primer, on the detection precision.

As shown in Table 11, a detection primer (VK1Mtg-pyren) to identify the gene polymorphism of VKORC1 was newly prepared. Regarding the detection primer, a primer in which Amino-modifier C6 dA (Glen Research) had been introduced in adenine at the second base from the 5' end, and furthermore, 6-fluorescein (Glen Research) had been introduced in the 5' end thereof, was purchased from Japan Bio Services, and the thus purchased primer was modified with pyrene in the amino group by using 1-pyrene butanoic acid succinimidyl ester (Invitrogen).

Regarding the competitive primer and the common primer, those synthesized by a usual synthesis method were purchased from Greiner Bio-One Japan. Regarding the competitive primer, a plurality of primers with variation of the position to introduce the mismatch base, were produced. The others are the same as those of the description in Comparative Example 1.

In Table 11, the polymorphic base recognition site is shown by bold, the mismatch base introduction site is underlined, and the pyrene-modified base is double underlined. In addition, "pyrene" denotes pyrene, "6-FAM" denotes the 6-FAM label, and the number on the right column denotes the sequence number corresponding to the sequence before labeling as shown in the Sequence Listing. The templates employed herein have the same

**[Table 11]**

| NAME OF OLIGONUCLEOTIDE | BASE SEQUENCE | |
|---|---|---|
| DETECTION PRIMER | | |
| VK1Wat-pyrene | 5'-6-FAM-TACCTCCCATCCTAGTCCAATG-3' | 10 |
| VK 1 Mtg-pyrene | 5'-6-FAM-TACCTCCCATCCTAGTCCATGA-3' | 11 |

| COMPETITIVE PRIMER | | |
|---|---|---|
| VK1M4t | 5'-ACCTCCCATCCTAGTCCTAGA-3' | 12 |
| VK1M5t | 5'-ACCTCCCATCCTAGTCTAAGA-3' | 13 |
| VK1MBt | 5'-ACCTCCCATCCTATTCCAAGA-3' | 14 |
| VK1M10a | 5'-ACCTCCCATCCAAGTCCAAGA-3' | 15 |
| VK1M17t | 5'-ACCTTCCATCCTAGTCCAAGA-3' | 16 |
| VK1W4t | 5'-ACCTCCCATCCTAGTCCTAGG-3' | 17 |
| VK1W5t | 5'-ACCTCCCATCCTAGTCTAAGG-3' | 18 |
| VK1W8t | 5'-ACCTCCCATCCTATTCCAAGG-3' | 19 |
| VK1W10a | 5'-ACCTCCCATCGAAGTCCAAGG-3' | 20 |
| VK1W17t | 5'-ACCTTCCATCCTAGTCCAAGG-3' | 21 |

| COMMON PRIMER | | |
|---|---|---|
| VK1R2 | 5'-AAAAGCAGGGCCTACG-3' | 9 |

### (Example 3)

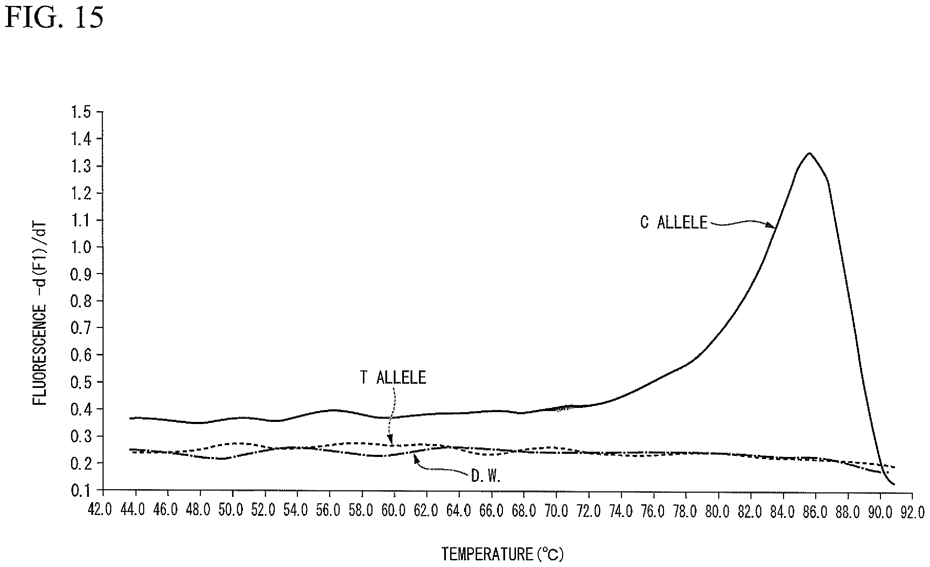

A reaction solution having a mixture of the C allele or the T allele of the *VKORC1* gene polymorphism as a template, VK1Wat-pyrene as a detection primer, VK1M4t as a competitive primer, and VK1R2 as a common primer, was prepared so that the composition would be as of Table 12. This reaction solution was set in the Real-time PCR system (a product manufactured by Roche, "Light Cycler") and held at 95°C for 1 minute, to effect denaturation of the antibody of the DNA polymerase. Then, two-step PCR consisting of 58°C for 20 seconds and 95°C for 5 seconds was run for 55 cycles. The melting curve analysis was carried out from 95°C to 40°C. A solution using distilled water (D.W.) as a template was employed as the negative control. The results are shown in FIG. 15.

**[Table 12]**

| | |
|---|---|
| 5X Colorless GoTaq Flexi Buffer (Promega) | 2.0 *µ*l |
| 25mM MgCl₂ (Promega) | 1.0 *µ*l |
| 2.5mM each dNTP (Promega) | 0.8 *µ*l |
| 2.5uM DETECTION PRIMER | 1.3 *µ*l |
| 2.5uM COMPETITIVE PRIMER | 1.3 *µ*l |
| 2. 5uM COMMON PRIMER | 1.3 *µ*l |
| 5U/ul GoTaq Hot Start Polymerase (Promega) | 0.1 *µ*l |
| 3ng/ul TEMPLATE (Coriell) | 1.0 *µ*l |
| DISTILLED WATER | BALANCE |
| TOTAL VOLUME OF REACTION SOLUTION | 10.0 *µ*l |

FIG. 15 shows the results of Example 3 expressed by negative first differential curves of the melting curves thereof.

In the case of using a set of the detection primer and the competitive primer of Example 3, similar results to those of Example 1 were obtained. Accordingly, it was suggested that, even though the mismatch base was located in the fourth base from the 3' end of the competitive primer, better C allele specificity was achieved similarly to the case of Example 1 where the mismatch base was located in the third base from the 3' end.

### (Example 4)

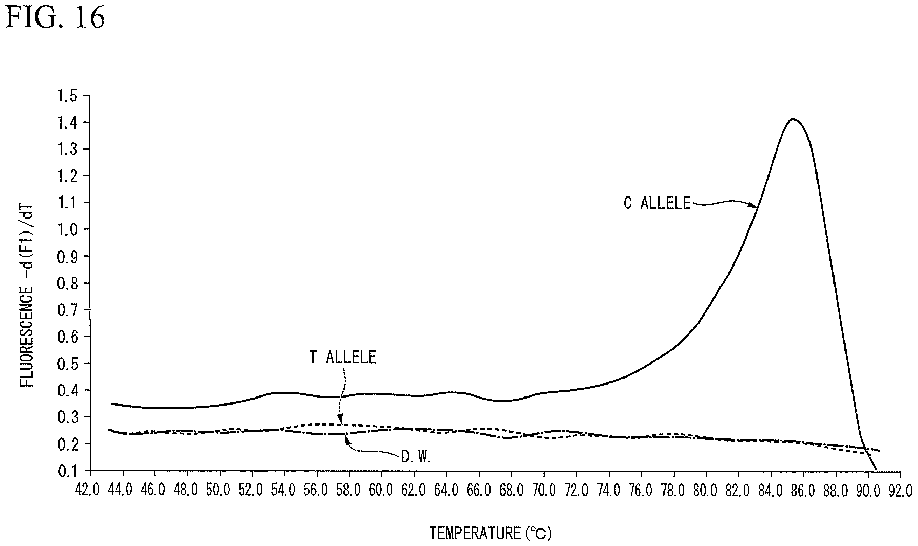

The reaction and the analysis were conducted in the same manner as for Example 3 except for using VK1M5t as a competitive primer. The results are shown in FIG. 16.

FIG. 16 shows the results of Example 4 expressed by negative first differential curves of the melting curves thereof.

In the case of using a set of the detection primer and the competitive primer of Example 4, similar results to those of Example 3 were obtained. Accordingly, it was suggested that, even though the mismatch base was located in the fifth base from the 3' end of the competitive primer, better C allele specificity was achieved similarly to the case of Example 1 where the mismatch base was located in the third base from the 3' end.

### (Example 5)

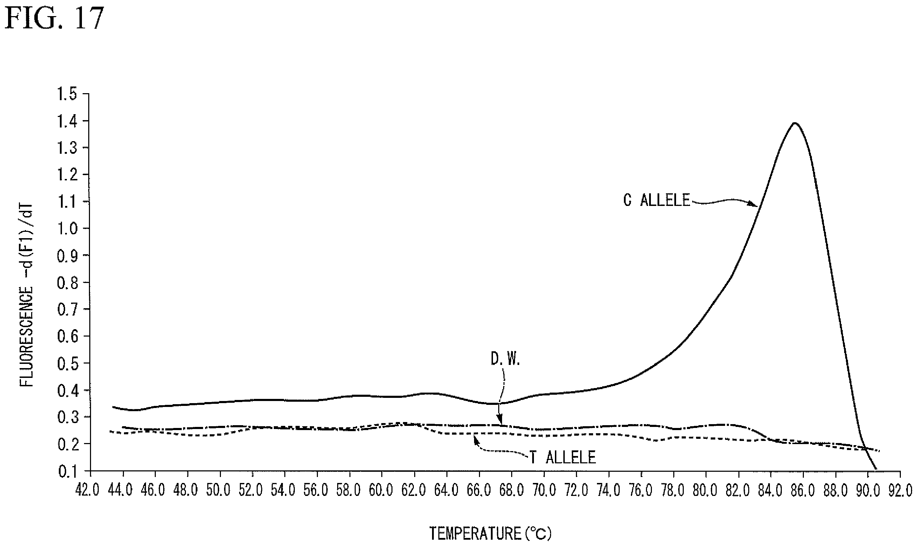

The reaction and the analysis were conducted in the same manner as for Example 3 except for using VK1M8t as a competitive primer. The results are shown in FIG. 17.

FIG. 17 shows the results of Example 5 expressed by negative first differential curves of the melting curves thereof.

In the case of using a set of the detection primer and the competitive primer of Example 5, similar results to those of Example 3 were obtained. Accordingly, it was suggested that, even though the mismatch base was located in the eighth base from the 3' end of the competitive primer, better C allele specificity was achieved similarly to the case of Example 1 where the mismatch base was located in the third base from the 3' end.

### (Example 6)

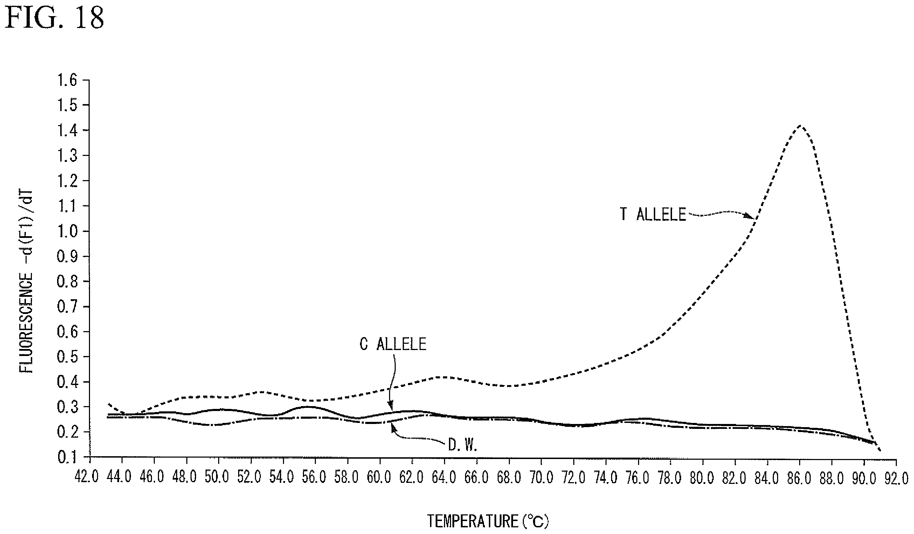

The reaction and the analysis were conducted in the same manner as for Example 3 except for using VK1Mtg-pyrene as a detection primer and VK1W4t as a competitive primer. The results are shown in FIG. 18.

FIG. 18 shows the results of Example 6 expressed by negative first differential curves of the melting curves thereof.

In the case of using a set of the detection primer and the competitive primer of Example 6, similar results to those of Example 2 were obtained. Accordingly, it was suggested that, even though a mismatch base had been introduced in the fourth base from the 3' end of the competitive primer in the primer set to detect the mutation, better T allele specificity was achieved.

### (Example 7)

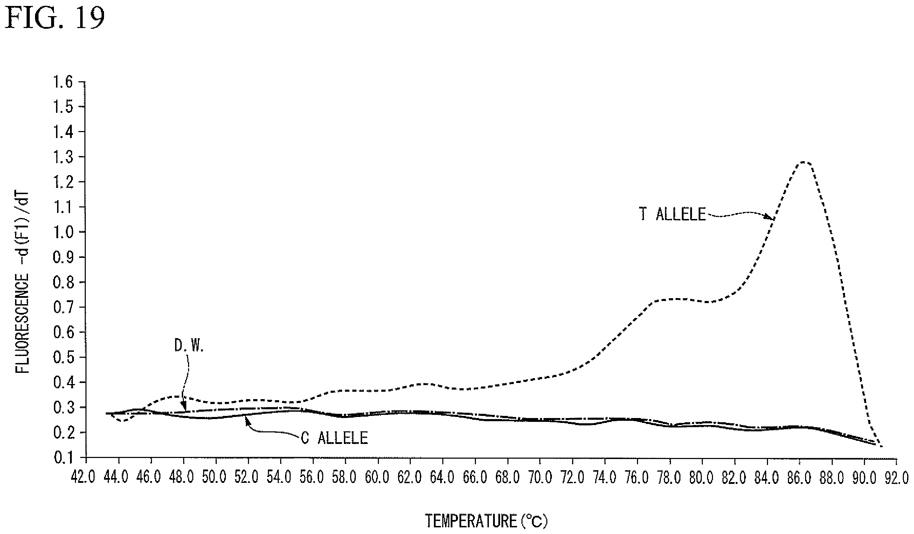

The reaction and the analysis were conducted in the same manner as for Example 6 except for using VK1W5t as a competitive primer. The results are shown in FIG. 19.

FIG. 19 shows the results of Example 7 expressed by negative first differential curves of the melting curves thereof.

In the case of using a set of the detection primer and the competitive primer of Example 7, similar results to those of Example 6 were obtained. Accordingly, it was suggested that, even though a mutation had been introduced in the fifth base from the 3' end of the competitive primer in the primer set to detect the mutation, better T allele specificity was achieved.

### (Example 8)

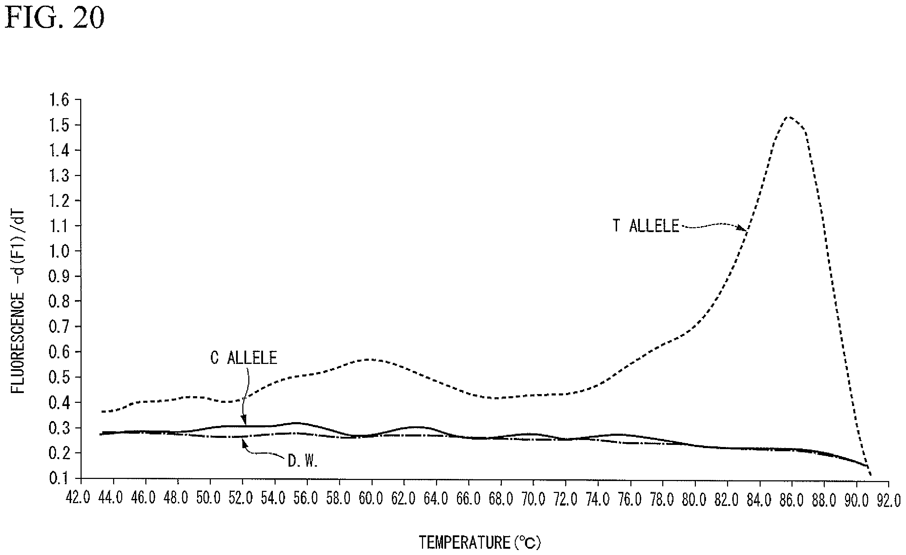

The reaction and the analysis were conducted in the same manner as for Example 7 except for using VK1W8t as a competitive primer. The results are shown in FIG. 20.

FIG. 20 shows the results of Example 8 expressed by negative first differential curves of the melting curves thereof.

In the case of using a set of the detection primer and the competitive primer of Example 8, similar results to those of Example 6 were obtained. Accordingly, it was suggested that, even though a mutation had been introduced in the eighth base from the 3' end of the competitive primer in the primer set to detect the mutation, better T allele specificity was achieved.

### (Examples 9 to 18)

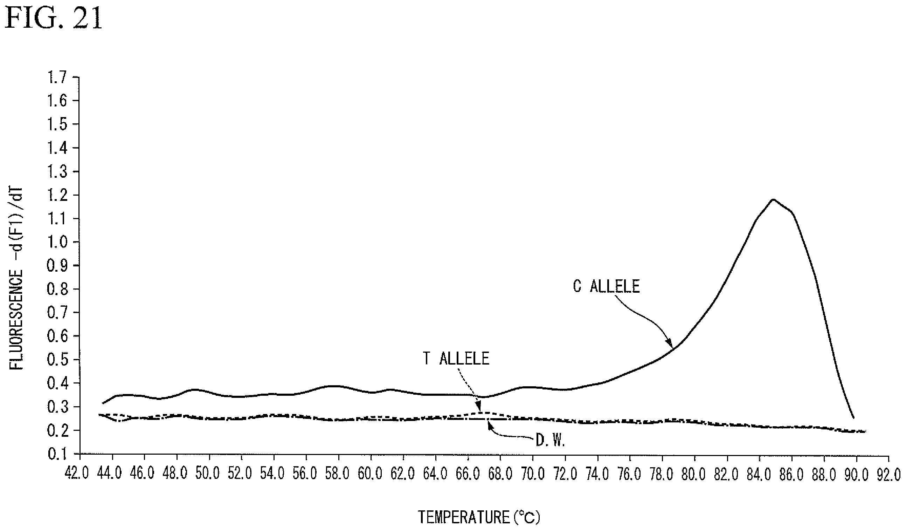

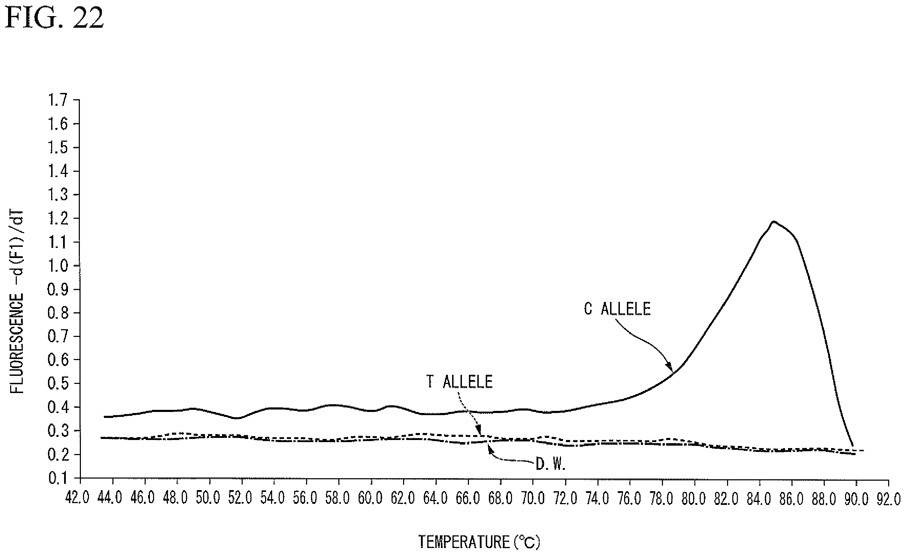

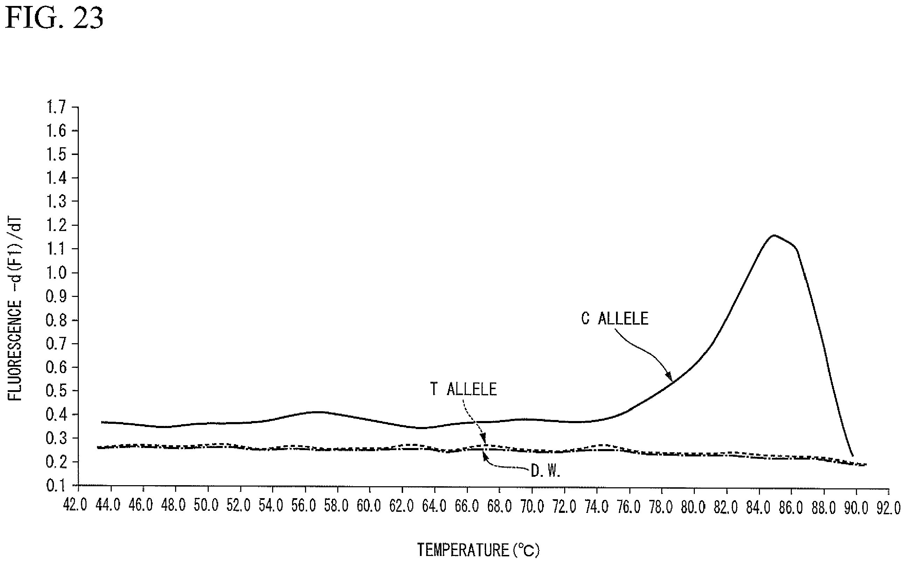

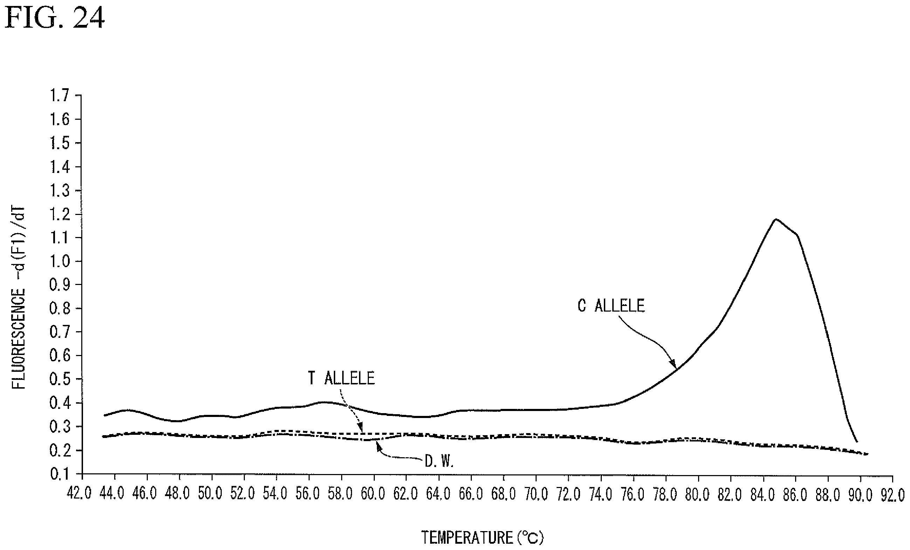

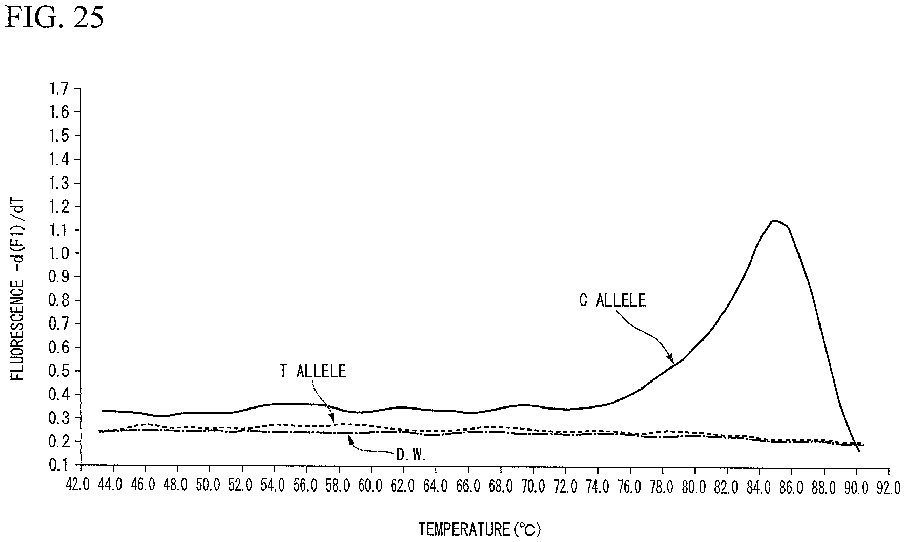

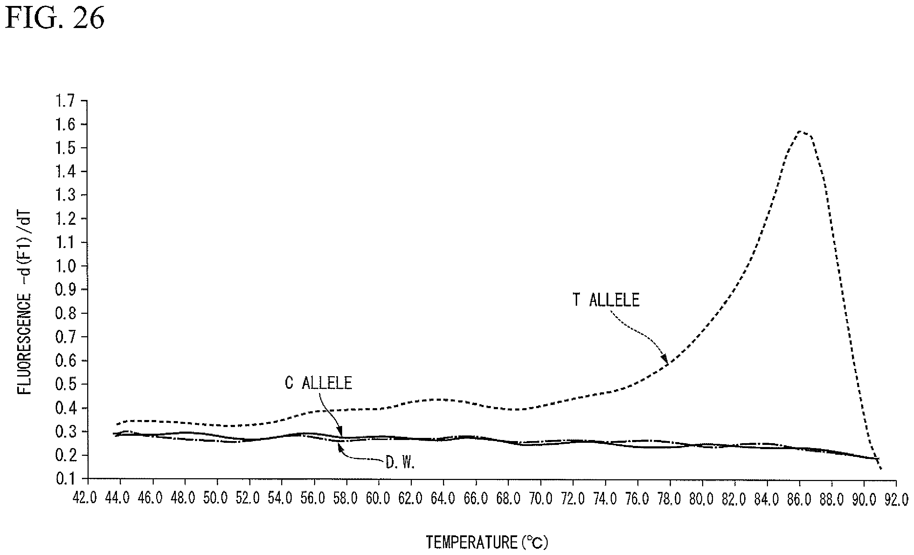

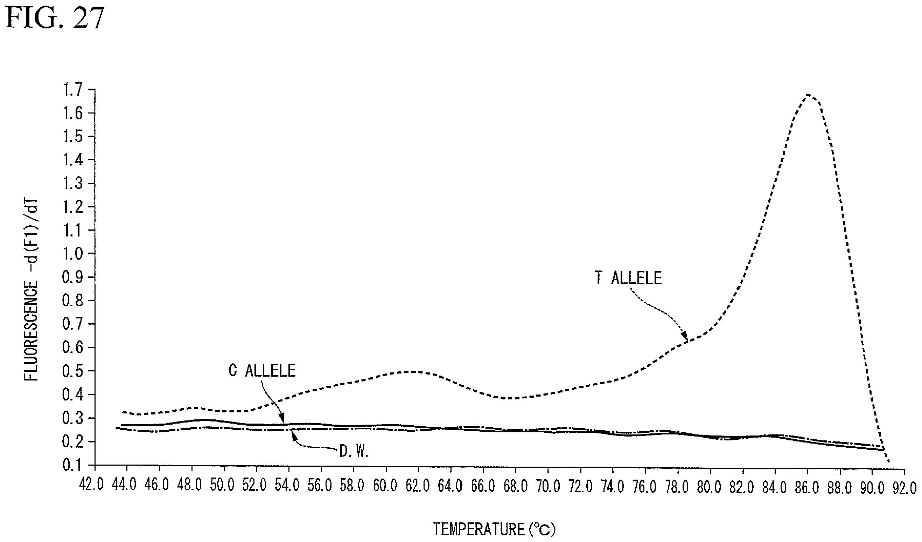

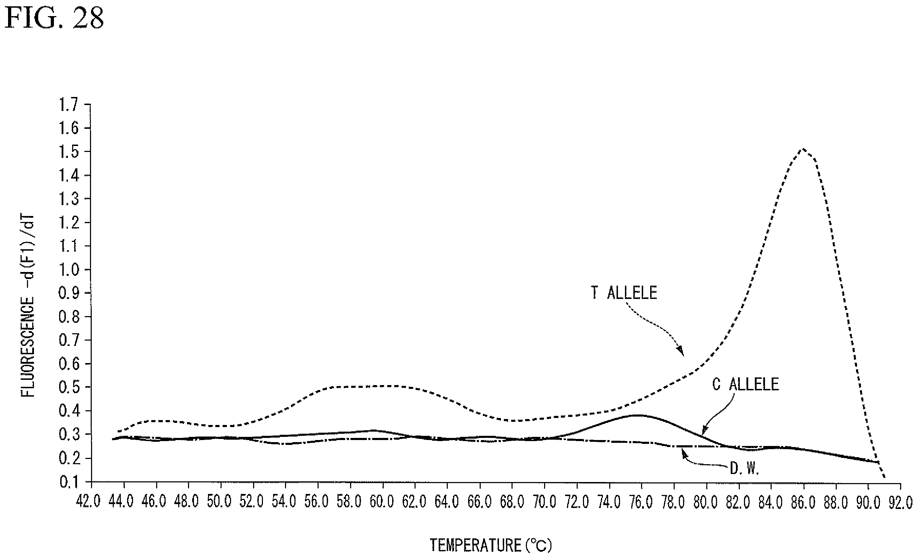

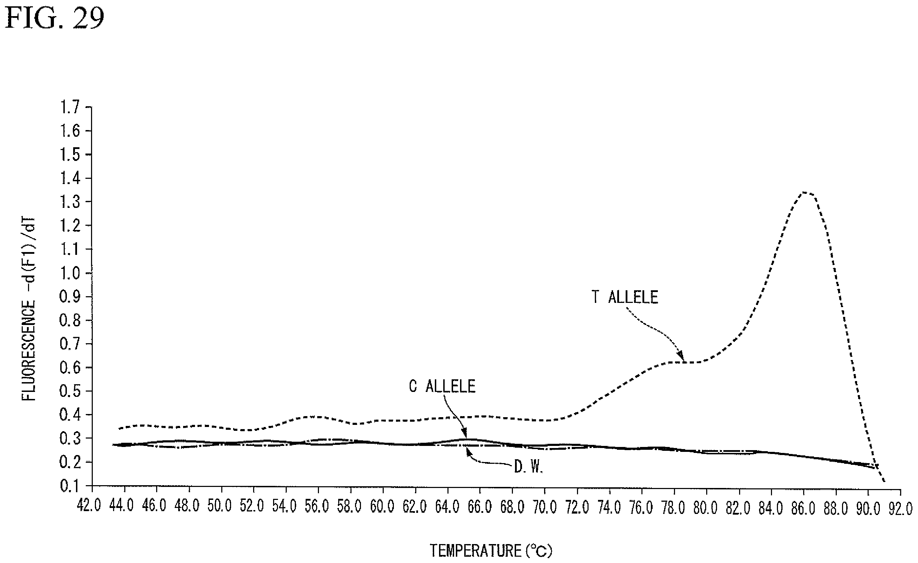

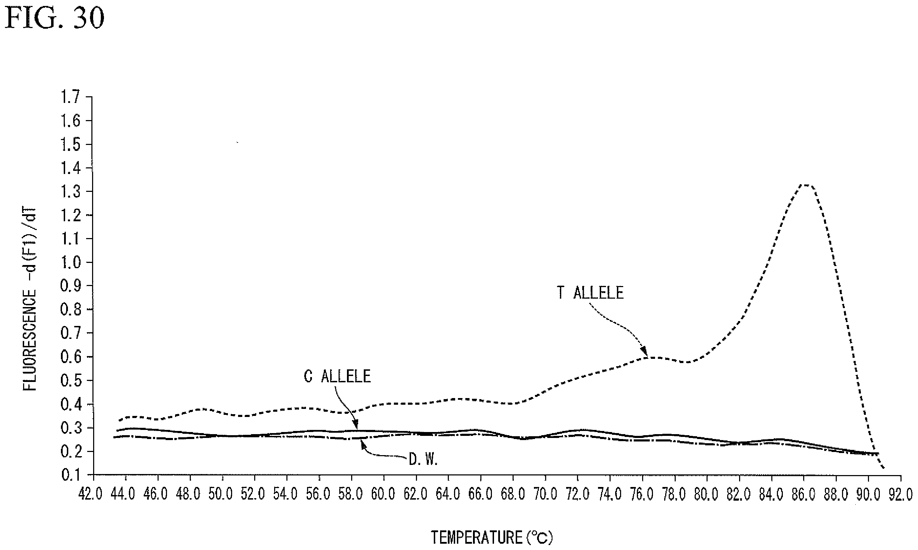

A reaction solution having a mixture of the C allele or the T allele of the *VKORC1* gene polymorphism as a template, a set of the detection primer and the competitive primer shown in Table 13 as primers, and VK1R2 as a common primer, was prepared so that the composition would be as of Table 13. This reaction solution was set in the Real-time PCR system (a product manufactured by Roche, "Light Cycler") and held at 95°C for 1 minute, to effect denaturation of the antibody of the DNA polymerase. Then, two-step PCR consisting of 62°C for 20 seconds and 95°C for 5 seconds was run for 55 cycles. The melting curve analysis was carried out from 95°C to 40°C. A solution using distilled water (D.W.) as a template was employed as the negative control. The results are shown in FIGs. 21 to 30.

**[Table 13]**

| | DETECTION PRIMER | COMPETITIVE PRIMER | RESULT |
|---|---|---|---|
| EXAMPLE 9 | VK1Wat-pyrene | VK1M4t | FIG. 21 |
| EXAMPLE 10 | VK1Wat-pyrene | VK1M5t | FIG. 22 |
| EXAMPLE 11 | VK1Wat-pyrene | VK1M8t | FIG. 23 |
| EXAMPLE 12 | VK1Wat-pyrene | VK1M10a | FIG. 24 |
| EXAMPLE 13 | VK1Wat-pyrene | VK1M17t | FIG. 25 |
| EXAMPLE 14 | VK1Mtg-pyrene | VK1W4t | FIG. 26 |
| EXAMPLE 15 | VK1Mtg-pyrene | VK1W5t | FIG. 27 |
| EXAMPLE 16 | VK1Mtg-pyrene | VK1W8t | FIG. 28 |
| EXAMPLE 17 | VK1Mtg-pyrene | VK1W10a | FIG. 29 |
| EXAMPLE 18 | VK1Mtg-pyrene | VK1W17t | FIG. 30 |

FIGs. 21 to 30 show the results of Examples 9 to 18 expressed by negative first differential curves of the melting curves thereof.

In the case of using a set of the detection primer and the competitive primer of Examples 9 to 13, similar results to those of Example 3 were obtained. In the case of using a set of the detection primer and the competitive primer of Examples 14 to 18, similar results to those of Example 6 were obtained.

From Examples 9 to 13, it was confirmed that the gene polymorphism was able to be detected with excellent precision in the case where the mismatch base of the detection primer and the mismatch base of the competitive primer were located within 17 bases from the base that can match to the polymorphic base.

In addition, from Examples 3 to 8, it was also confirmed that the gene polymorphism was able to be detected with excellent precision, even under a condition of low annealing temperature, in the case where such mismatch bases were located within 8 bases therefrom.

In Examples 19 to 27, similarly to the above-mentioned cases, a gene polymorphism (1173C>T) in the position 1173 of the intron 1 region of the vitamin K epoxide reductase complex 1 (VKORC1), which is a gene related to the optimum dose of warfarin, was used as a detection target, so as to investigate the influence of the difference of the chain length between the competitive primer and the detection primer, on the detection precision.

As shown in Table 14, a detection primer (VK1Wat-P-FAM1) to identify the gene polymorphism of VKORC1 was newly prepared. Regarding the detection primer, a primer in which Amino-modifier C6 dC (Glen Research) had been introduced in cytosine at the fourth base from the 5' end, and furthermore, fluorescein dT (Glen Research) had been introduced in thymine at the sixth base from the 5' end, was purchased from Japan Bio Services, and the thus purchased primer was modified with pyrene in the amino group by using 1-pyrene butanoic acid succinimidyl ester (Invitrogen).

Regarding the competitive primer and the common primer shown in Table 14, those synthesized by a usual synthesis method were purchased from Greiner Bio-One Japan. Regarding the competitive primer, a plurality of primers with variation of the chain length, were produced.

In Table 14, the position 1173 and the polymorphic base recognition site are shown by bold, and the mismatch base introduction site is underlined. In addition, "FAMdT" denotes the fluorescein dT, "PyrendC" denotes the Amino-modifier C6 dC labeled with pyrene, and the number on the right column denotes the sequence number corresponding to the sequence before labeling as shown in the Sequence Listing. The templates employed herein have the same sequences as those of Table 9.

### (Example 19 to 27)

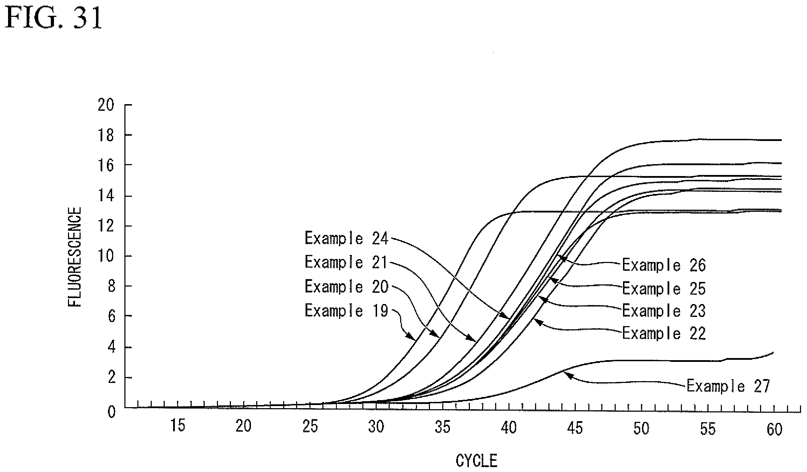

A reaction solution having a mixture of the C allele or the T allele of the VKORC 1 gene polymorphism as a template, a set of the detection primer and the competitive primer shown in Table 15 as primers, and VK1R2 as a common primer, was prepared so that the composition would be as of Table 10. This reaction solution was set in the Real-time PCR system (a product manufactured by Roche, "Light Cycler 480") and held at 95°C for 1 minute, to effect denaturation of the antibody of the DNA polymerase. Then, two-step PCR consisting of 64°C for 30 seconds and 95°C for 5 seconds was run for 60 cycles. The results obtained by reactions with use of the C allele as a template are shown in FIG. 31.

**[Table 14]**

| NAME OF OLIGONUCLEOTIDE | BASE SEQUENCE | |
|---|---|---|
| DETECTION PRIMER | | |
| VK1Wat-P-FAM1 | 5'-CGA-(Pyrene dC)-C-(FAM dT)-CCCATCCTAGTCCAATG-3' | 22 |

| COMPETITIVE PRIMER | | |
|---|---|---|
| VK1Mtg-23 | 5'-CGACCTCCCATCCTAGTCCATGA-3' | 23 |
| VK1Mtg-24 | 5'-CCGACCTCCCATCCTAGTCCATGA-3' | 24 |
| VK1Mtg-25 | 5'-CCCGACCTCCCATCCTAGTCCATGA-3' | 25 |
| VK1Mtg-26 | 5'-CCCCGACCTCCCATCCTAGTCCATGA-3' | 26 |
| VK1Mtg-27 | 5'-TCCCCGACCTCCCATCCTAGTCCATGA-3' | 27 |
| VK1Mtg-31 | 5'-CTGTTCCCCGACCTCCCATCCTAGTCCATGA-3' | 28 |
| VK1Mtg-35 | 5'-TCCTCTGTTCCCCGACCTCCCATCCTAGTCCATGA-3' | 29 |
| VK1Mtg-39 | 5'-GCTATCCTCTGTTCCCCGACCTCCCATCCTAGTCCATGA-3' | 30 |
| VK1Mtg-43 | 5'-CTGGGCTATCCTCTGTTCCCCGACCTCCCGATCCTAGTCCATGA-3' | 31 |

| COMMON PRIMER | | |
|---|---|---|
| VK1R2 | 5'-AAAAGCAGGGCCTACG-3' | 9 |

**[Table 15]**

| | DETECTION PRIMER | COMPETITIVE PRIMER | RESULT |
|---|---|---|---|
| EXAMPLE 19 | VK1Wat-P-FAM1 | VK1Mtg-23 | FIG. 31 |
| EXAMPLE 20 | VK1Wat-P-FAM1 | VK1Mtg-24 | FIG. 31 |
| EXAMPLE 21 | VK1Wat-P-FAM1 | VK1Mtg-25 | FIG. 31 |
| EXAMPLE 22 | VK1Wat-P-FAM1 | VK1Mtg-26 | FIG. 31 |
| EXAMPLE 23 | VK1Wat-P-FAM1 | VK1Mtg-27 | FIG. 31 |
| EXAMPLE 24 | VK1Wat-P-FAM1 | VK1Mtg-31 | FIG. 31 |
| EXAMPLE 25 | VK1Wat-P-FAM1 | VK1Mtg-35 | FIG. 31 |
| EXAMPLE 26 | VK1Wat-P-FAM1 | VK1Mtg-39 | FIG. 31 |
| EXAMPLE 27 | VK1Wat-P-FAM1 | VK1Mtg-43 | FIG. 31 |

As shown in FIG. 31, it was confirmed that the gene polymorphism was able to be detected with excellent precision when using the set of the detection primer and the competitive primer of Examples 19 to 27.

In Examples 21 to 26 using the competitive primer which was 2 bases to 16 bases longer than the detection primer, it was confirmed that the reactivity was better than Example 27 using the competitive primer which was 20 bases longer than the detection primer.

Furthermore, in Example 19 and Example 20 using the competitive primer having the same chain length as that of, or one base longer than, the detection primer, it was confirmed that the reactivity was better than Examples 21 to 26 using the competitive primer which was 2 bases to 16 bases longer than the detection primer.

In Examples 28 to 35, similarly to the above-mentioned cases, a gene polymorphism (1173C>T) in the position 1173 of the intron 1 region of the vitamin K epoxide reductase complex 1 (VKORC1), which is a gene related to the optimum dose of warfarin, was used as a detection target, so as to investigate the influence of the second mismatch bases introduced in the competitive primer and the detection primer, on the detection precision.

As shown in Table 16, the detection primers (VK1Wat-P-FAM2 to 8) to identify the gene polymorphism of VKORC1 were newly prepared. Regarding the detection primers, those in which Amino-modifier C6 dC (Glen Research) had been introduced in cytosine at the fourth base from the 5' end, and furthermore, fluorescein dT (Glen Research) had been introduced in thymine at the sixth base thereof, were purchased from Japan Bio Services, and the thus purchased ones were modified with pyrene in the amino group by using 1-pyrene butanoic acid succinimidyl ester (Invitrogen).

Regarding the competitive primer and the common primer shown in Table 16, those synthesized by a usual synthesis method were purchased from Greiner Bio-One Japan. Regarding the competitive primer, a plurality of primers with variation of the position to introduce the second mismatch base, were produced.

In Table 16, the position 1173 and the polymorphic base recognition site are shown by bold, and the mismatch base introduction site is underlined. In addition, "FAMdT" denotes fluorescein dT, "PyrendC" denotes the Amino-modifier C6 dC labeled with pyrene label, and the number on the right column denotes the sequence number corresponding to the sequence before labeling as shown in the Sequence Listing. The templates employed herein have the same sequences as those of Table 9.

### (Example 28 to 35)

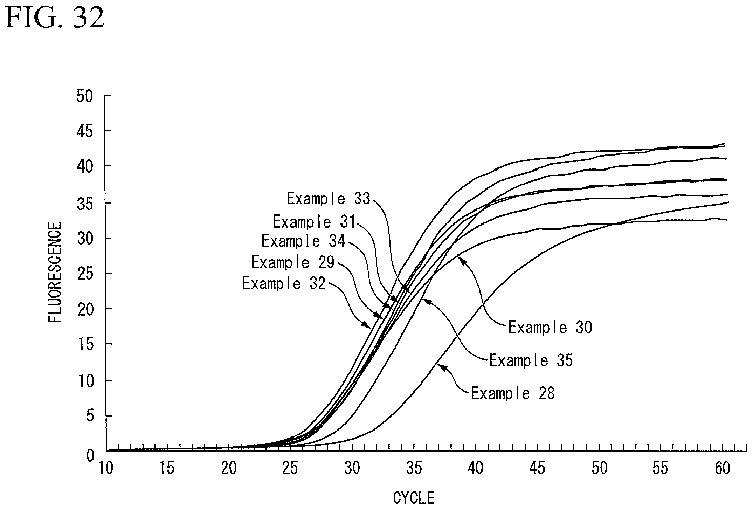

A reaction solution having a mixture of the C allele or the T allele of the VKORC 1 gene polymorphism as a template, a set of the detection primer and the competitive primer shown in Table 17 as primers, and VK1R2 as a common primer, was prepared so that the composition would be as of Table 10. This reaction solution was set in the Real-time PCR system (a product manufactured by Roche, "Light Cycler 480") and held at 95°C for 1 minute, to effect denaturation of the antibody of the DNA polymerase. Then, two-step PCR consisting of 64°C for 30 seconds and 95°C for 5 seconds was run for 60 cycles. The results obtained by reactions with use of the C allele as a template are shown in FIG. 32.

**[Table 16]**

| NAME OF OLIGONUCLEOTIDE | BASE SEQUENCE | |
|---|---|---|
| DETECTION PRIMER | | |
| VK1Wat-P-FAM1 | 5'-CGA-(Pyrene dC)-C-(FAM dT)-CCCATCCTAGTCCAATG-3' | 22 |
| VK1Wat-P-FAM2 | 5'-CGA-(Pyrene dC)-C-(FAM dT)-CCCATTCTAGTCCAATG-3' | 31 |
| VK1Wat-P-FAM3 | 5'-CGA-(Pyrene dC)-C-(FAM dT)-CCCGTCCTAGTCCAATG-3' | 32 |
| VK1Wat-P-FAM4 | 5'-CGA-(Pyrene dC)-C-(FAM dT)-CTCATCCTAGTCCAATG-3' | 33 |
| VK1Wat-P-FAM5 | 5'-CGA-(Pyrene dC)-T-(FAM dT)-CCCATCCTAGTCCAATG-3' | 34 |
| VK1Wat-P-FAM6 | 5'-CTA-(Pyrene dC)-C-(FAM dT)-CCCATCCTAGTCC)AATG-3' | 35 |
| VK1Wat-P-FAM7 | 5'-CGA-(Pyrene dC)-C-(FAM dT)-CCGATCCCAGTCCAATG-3' | 36 |
| VK1Wat-P-FAM8 | 5'-CGA-(Pyrene dC)-C-(FAM dT)-CCCATCCTATTCCAATG-3' | 37 |

| COMPETITIVE PRIMER | | |
|---|---|---|
| VK1Mtg-23-1 | 5'-CGACCTCCCATCCTAGTCCATGA-3' | 38 |
| VK1Mtg-23-2 | 5'-CGACCTCCCATACTAGTCCATGA-3' | 39 |
| VK1Mtg-23-3 | 5'-CGACCTCCCCTCCTAGTCCATGA-3' | 40 |
| VK1Mtg-23-4 | 5'-CGACCTCACATCCTAGTCCATGA-3' | 41 |
| VK1Mtg-23-5 | 5'-CGACATCCCATCCTAGTCCATGA-3' | 42 |
| VK1Mtg-23-6 | 5'-CAACCTCCCATCCTAGTCCATGA-3' | 43 |
| VK1Mtg-23-7 | 5'-CGACCTCCCATCCGAGTCCATGA-3' | 44 |
| VK1Mtg-23-8 | 5'-CGACCTCCCATCCTAACCATGA-3' | 45 |

| COMMON PRIMER | | |
|---|---|---|
| VK1R2 | 5'-AAAAGCAGGGCCTACG-3' | 9 |

**[Table 17]**

| | DETECTION PRIMER | COMPETITIVE PRIMER | RESULT |
|---|---|---|---|
| EXAMPLE 28 | VK1Wat-P-FAM1 | VK1Mtg-23-1 | FIG. 32 |
| EXAMPLE 29 | VK1Wat-P-FAM2 | VK1Mtg-23-2 | FIG. 32 |
| EXAMPLE 30 | VK1Wat-P-FAM3 | VK1Mtg-23-3 | FIG. 32 |
| EXAMPLE 31 | VK1Wat-P-FAM4 | VK1Mtg-23-4 | FIG. 32 |
| EXAMPLE 32 | VK1Wat-P-FAM5 | VK1Mtg-23-5 | FIG. 32 |
| EXAMPLE 33 | VK1Wat-P-FAM6 | VK1Mtg-23-6 | FIG. 32 |
| EXAMPLE 34 | VK1Wat-P-FAM7 | VK1Mtg-23-7 | FIG. 32 |
| EXAMPLE 35 | VK1Wat-P-FAM8 | VK1Mtg-23-8 | FIG. 32 |

As shown in FIG. 32, it was confirmed that the gene polymorphism was able to be detected with excellent precision when using the set of the detection primer and the competitive primer of Examples 28 to 35.

In Examples 29 to 35 using the set of the detection primer and the competitive primer in which the second mismatch bases were 7 bases or more away from the 3' end, it was confirmed that the reactivity was better than Example 28 in which no second mismatch base was introduced.

Of these, in Examples 29 to 34 using the set of the detection primer and the competitive primer in which the second mismatch bases were 9 bases or more away from the 3' end, it was confirmed that the reactivity was much better.

### INDUSTRIAL APPLICABILITY

The method of detecting a target base sequence using the competitive primer of the present invention is excellent in the genotype identification precision, and hence is applicable to the fields of clinical test and the like, particularly to the fields of single nucleotide polymorphism and somatic mutation.

## Claims

1. A method of detecting a target base sequence having a polymorphic base comprising
(a) a step of adding the following primers to a nucleic acid sample having a target nucleic acid that comprises a base sequence including the target base sequence:
at least one type of detection primer that is complementary to said target base sequence,
at least one type of competitive primer that is complementary to said target base sequence as well as being capable of annealing to a target nucleic acid, in a competitive manner against said detection primer, and
at least one type of common primer,
(b) a step of annealing said detection primer and said competitive primer to said target nucleic acid in a competitive manner with use of the target base sequence having the polymorphic base in said nucleic acid sample as a template, thereby causing an extension reaction to synthesize an extension product A,
(c) a step of annealing said common primer to said extension product A obtained in said step (b) or in the following step (d), thereby causing an extension reaction to synthesize an extension product B,
(d) a step of annealing said detection primer or said competitive primer to the extension product B obtained in said step (c), thereby synthesizing the extension product A, and
(e) a step of detecting the extension product A or the extension product B, wherein
said detection primer has a match base that is complementary to the polymorphic base of the target sequence, and has at least one first mismatch base that is not complementary to a base of the target sequence other than the polymorphic base;
said competitive primer has a mismatch base that is not complementary to the polymorphic base of the target sequence, and has at least one first mismatch base and optionally at least one second mismatch base that are not complementary to a base of the target sequence other than the polymorphic base;
the position of the at least one first mismatch base of said detection primer is different from the position of the at least one first mismatch base of said competitive primer that is not complementary to the base of the target sequence other than the polymorphic base;
the detection primer has the match base that is complementary to the polymorphic base of the target sequence, at the 3' end or the second base from the 3' end;
said common primer is capable of making a primer pair with said detection primer or said competitive primer to amplify said target nucleic acid;
a difference in a chain length between said competitive primer and said detection primer is within 16 bases;
when present, the second mismatch base of said competitive primer is located 7 bases or more away from the 3' end to the 5' side.

2. The method of detecting a target base sequence according to claim 1, wherein
in said detection primer and said competitive primer,
the first mismatch base that is not complementary to a base of the target sequence other than the polymorphic base is located within 17 bases from the match base that is complementary to the polymorphic base of the target sequence in the case of said detection primer, and from the mismatch base that is not complementary to the polymorphic base of the target sequence in the case of said competitive primer, and the position of each mismatch base of each primer is different.

3. The method of detecting a target base sequence according to claim 1 or 2, wherein
said detection primer further has at least one second mismatch base that is not complementary to a base of the target sequence other than the polymorphic base,
said competitive primer has the second mismatch base,
the first mismatch base of the detection primer is located within 6 bases from the 3' end,
the second mismatch base of the detection primer is located 7 bases or more away from the 3' end to the 5' side, and
the second mismatch base of said detection primer and the second mismatch base of said competitive primer are different from each other.

4. The method of detecting a target base sequence according to claim 3, wherein the position of said second mismatch base is the same for both said detection primer and said competitive primer.

5. The method of detecting a target base sequence according to any one of claims 1 to 4, wherein said steps (b) to (d) are steps performed by any one selected from a group consisting of polymerase chain reaction, loop-mediated isothermal amplification, nucleic acid sequence based amplification, isothermal and chimerical primer-initiated amplification of nucleic acids, transcription reverse-transcription concerted reaction, strand displacement amplification, transcription mediated amplification, smart amplification process, recombinase polymerase amplification, and helicase-dependent amplification.

6. The method of detecting a target base sequence according to any one of claims 1 to 5, wherein at least one of said detection primer, said competitive primer, and said common primer is labeled.

7. The method of detecting a target base sequence according to claim 6, wherein a labeling substance for use in said labeling is at least one selected from a group consisting of a fluorophore and an energy absorbing material.

8. The method of detecting a target base sequence according to claim 6 or claim 7, wherein, said detection primer and said competitive primer are respectively labeled with different types of labeling substances, and said step (e) is a step of separately detecting the extension product from said detection primer and the extension product from said competitive primer.

9. The method of detecting a target base sequence according to any one of claims 6 to 8, wherein said step (e) is a step to be performed simultaneously with said steps (b) to (d), as well as being a step of detecting a state where the extension product from a labeled primer forms a double strand.

10. The method of detecting a target base sequence according to any one of claims 6 to 8, wherein said step (e) is a step to be performed after said step (d), as well as being a step of performing the detection with use of a melting curve or an amplification curve of said extension product.

11. The method of detecting a target base sequence according to any one of claims 6 to 10, wherein said step (e) is a step of performing the detection through use of a QP (Quenching Probe/Primer) method.

12. A kit for use in the method of detecting a target base sequence having a polymorphic base comprising at least one type of detection primer that is complementary to said target base sequence,
at least one type of competitive primer that is complementary to said target base sequence as well as being capable of annealing to target nucleic acid in a competitive manner against said detection primer, and
at least one type of common primer, wherein
said detection primer has a match base that is complementary to the polymorphic base of the target sequence, and has at least one first mismatch base that is not complementary to a base of the target sequence other than the polymorphic base of said target sequence;
said competitive primer has a mismatch base that is not complementary to the polymorphic base of the target sequence, and has at least one first mismatch base and optionally at least one second mismatch base that are not complementary to a base of the target sequence other than the polymorphic base;
the position of the at least one first mismatch base of said detection primer is different from the position of the at least one first mismatch base of said competitive primer that is not complementary to the base of the target sequence other than the polymorphic base;
the detection primer has the match base that is complementary to the polymorphic base of the target sequence, at the 3' end or the second base from the 3' end;
said common primer is capable of making a primer pair with said detection primer or said competitive primer to amplify said target nucleic acid;
a difference in a chain length between said competitive primer and said detection primer is within 16 bases;
when present, the second mismatch base of said competitive primer is located 7 bases or more away from the 3' end to the 5' side.

## Patentansprüche

1. Verfahren zum Detektieren einer Zielbasensequenz, die eine polymorphe Base aufweist, umfassend
(a) einen Schritt des Addierens der folgenden Primer an eine Nukleinsäureprobe, die eine Zielnukleinsäure aufweist, die eine Basensequenz, die die Zielbasensequenz einschließt, umfasst:
mindestens einen Typ Detektionsprimer, der zu der Zielbasensequenz komplementär ist,
mindestens einen Typ von kompetitivem Primer, der zu der Zielbasensequenz komplementär sowie in der Lage ist, eine Zielnukleinsäure auf kompetitive Weise gegen den Detektionsprimer zu annealen; und
mindestens einen Typ von gewöhnlichem Primer,
(b) einen Schritt des Annealens des Detektionsprimers und des kompetitiven Primers an die Zielnukleinsäure auf kompetitive Weise unter Verwendung der Zielbasensequenz, die die polymorphe Base in der Nukleinsäureprobe aufweist, als Matrize, wodurch eine Extensionsreaktion zum Synthetisieren eines Extensionsprodukts A verursacht wird,
(c) einen Schritt des Annealens des gewöhnlichen Primers an das in Schritt (b) oder dem folgenden Schritt (d) erhaltene Extensionsprodukt A, wodurch eine Extensionsreaktion zum Synthetisieren eines Extensionsprodukts B verursacht wird,
(d) einen Schritt des Annealens des Detektionsprimers oder des kompetitiven Primers an das in Schritt (c) erhaltene Extensionsprodukt B, wodurch das Extensionsprodukt A synthetisiert wird, und
(e) einen Schritt des Detektierens des Extensionsprodukts A oder des Extensionsprodukts B, wobei
der Detektionsprimer eine Match-Base aufweist, die zu der polymorphen Base der Zielsequenz komplementär ist und mindestens eine erste Mismatch-Base aufweist, die zu einer Base der Zielsequenz, außer der polymorphen Base, nicht komplementär ist;
wobei der kompetitive Primer eine Mismatch-Base aufweist, die zu der polymorphen Base der Zielsequenz nicht komplementär ist und mindestens eine erste Mismatch-Base und wahlweise mindestens eine zweite Mismatch-Base aufweist, die zu einer Base der Zielsequenz, außer der polymorphen Base ist, nicht komplementär sind;
die Position der mindestens einen ersten Mismatch-Base des Detektionsprimers von der Position der mindestens einen ersten Mismatch-Base des kompetitiven Primers, der zu der Base der Zielsequenz, außer der polymorphen Base, nicht komplementär ist, verschieden ist;
der Detektionsprimer die Match-Base, die zu der polymorphen Base der Zielsequenz komplementär ist, am 3'-Ende oder der zweiten Base vom 3'-Ende aufweist;
wobei der gewöhnliche Primer in der Lage ist, ein Primerpaar mit dem Detektionsprimer oder dem kompetitiven Primer zum Amplifizieren der Zielnukleinsäure zu erzeugen;
ein Unterschied einer Kettenlänge zwischen dem kompetitiven Primer und dem Detektionsprimer innerhalb von 16 Basen liegt;
die zweite Mismatch-Base, liegt sie vor, des zweiten kompetitiven Primers 7 Basen oder mehr von dem 3'-Ende zur 5'-Seite positioniert ist.

2. Verfahren zum Detektieren einer Zielbasensequenz nach Anspruch 1, wobei
in dem Detektionsprimer und dem kompetitiven Primer
die erste Mismatch-Base, die zu einer Base der Zielsequenz außer der polymorphen Base nicht komplementär ist, innerhalb von 17 Basen von der Match-Base, die zu der polymorphen Base der Zielsequenz komplementär ist, im Falle des Detektionsprimers, und von der Mismatch-Base, die zu der polymorphen Base der Zielsequenz nicht komplementär ist, im Falle des kompetitiven Primers positioniert ist und die Position jeder Mismatch-Base jedes Primers verschieden ist.

3. Verfahren zum Detektieren einer Zielbasensequenz nach Anspruch 1 oder 2, wobei der Detektionsprimer ferner mindestens eine zweite Mismatch-Base aufweist, die zu einer Base der Zielsequenz, außer der polymorphen Base, nicht komplementär ist,
der kompetitive Primer die zweite Mismatch-Base aufweist,
die erste Mismatch-Base des Detektionsprimers innerhalb von 6 Basen von dem 3'-Ende positioniert ist,
die zweite Mismatch-Base des Detektionsprimers 7 Basen oder mehr von dem 3'-Ende zur 5'-seite entfernt liegt, und
die zweite Mismatch-Base des Detektionsprimers und die zweite Mismatch-Base des kompetitiven Primers voneinander verschieden sind.

4. Verfahren zum Detektieren einer Zielbasensequenz nach Anspruch 3, wobei die Position der zweiten Mismatch-Base für beide, den Detektionsprimer und den kompetitiven Primer, dieselbe ist.

5. Verfahren zum Detektieren einer Zielbasensequenz nach einem der Ansprüche 1 bis 4, wobei die Schritte (b) bis (d) Schritte sind, die durch irgendeine ausgeführt werden ausgewählt aus der Gruppe bestehend aus Polymerasekettenreaktion, schleifenvermittelter isothermischer Amplifikation, Amplifikation auf der Basis von Nukleinsäure, von isothermischem und chimärem Primer ausgelöster Amplifikation von Nukleinsäuren, durch transkriptionsreverse Transkription konzertierter Reaktion, Strang-Displacement-Amplifikation, transkriptionsvermittelter Amplifikation, Smart-Amplifikationsverfahren, Rekombinase-Polymerase-Amplifikation und helikaseabhängiger Amplifikation.

6. Verfahren zum Detektieren einer Zielbasensequenz nach einem der Ansprüche 1 bis 5, wobei mindestens einer von dem Detektionsprimer, dem kompetitiven Primer und dem gewöhnlichen Primer markiert ist.

7. Verfahren zum Detektieren einer Zielbasensequenz nach Anspruch 6, wobei eine Markiersubstanz zur Verwendung beim Markieren mindestens eine ist ausgewählt aus einer Gruppe bestehend aus einem Fluorphor und einem Energie absorbierenden Material.

8. Verfahren zum Detektieren einer Zielbasensequenz nach Anspruch 6 oder Anspruch 7, wobei der Detektionsprimer und der kompetitive Primer jeweils mit verschiedenen Typen von Markiersubstanzen markiert sind und der Schritt (e) ein Schritt des getrennten Detektierens des Extensionsprodukts von dem Detektionsprimer und dem Extensionsprodukt von dem kompetitiven Primer ist.

9. Verfahren zum Detektieren einer Zielbasensequenz nach einem der Ansprüche 6 bis 8, wobei der Schritt (e) ein Schritt ist, der gleichzeitig mit den Schritten (b) bis (d) durchgeführt werden soll, sowie ein Schritt des Detektierens eines Zustands ist, wobei das Extensionsprodukt aus einem markierten Primer einen Doppelstrang bildet.

10. Verfahren zum Detektieren einer nach einem der Ansprüche 6 bis 8, wobei der Schritt (c) ein Schritt ist, der nach (d) durchgeführt werden soll, sowie ein Schritt des Ausführens der Detektion unter Anwendung einer Schmelzkurve oder einer Amplifikationskurve des Extensionsprodukts ist.

11. Verfahren zum Detektieren einer Zielbasensequenz nach einem der Ansprüche 6 bis 10, wobei der Schritt (c) ein Schritt des Ausführens der Detektion durch Verwendung eines QP-(Quenchsonden-/Primer-) Verfahrens ist.

12. Kit zur Verwendung bei dem Verfahren zum Detektieren einer Zielbasensequenz, die eine polymorphe Base aufweist, umfassend mindestens einen Typ Detektionsprimer, der zu der Zielbasensequenz komplementär ist,
mindestens einen Typ von kompetitivem Primer, der zu der Zielbasensequenz komplementär sowie in der Lage ist, eine Zielnukleinsäure auf kompetitive Weise gegen den Detektionsprimer zu annealen; und
mindestens einen Typ gewöhnlicher Primer, wobei
der Detektionsprimer eine Match-Base aufweist, die zu der polymorphen Base der Zielsequenz komplementär ist und mindestens eine erste Mismatch-Base aufweist, die zu einer Base der Zielsequenz, außer der polymorphen Base, nicht komplementär ist;
der kompetitive Primer eine Mismatch-Base aufweist, die zu der polymorphen Base der Zielsequenz nicht komplementär ist und mindestens eine erste Mismatch-Base und wahlweise mindestens eine zweite Mismatch-Base aufweist, die zu einer Base der Zielsequenz, außer der polymorphen Base, nicht komplementär sind;
die Position der mindestens einen ersten Mismatch-Base des Detektionsprimers von der Position der mindestens einen ersten Mismatch-Base des kompetitiven Primers, der zu der Base der Zielsequenz, außer der polymorphen Base, nicht komplementär ist, verschieden ist;
der Detektionsprimer die Match-Base, die zu der polymorphen Base der Zielsequenz komplementär ist, am 3'-Ende oder der zweiten Base vom 3'-Ende aufweist;
der gewöhnliche Primer in der Lage ist, ein Primerpaar mit dem Detektionsprimer oder dem kompetitiven Primer zum Amplifizieren der Zielnukleinsäure zu erzeugen;
ein Unterschied einer Kettenlänge zwischen dem kompetitiven Primer und dem Detektionsprimer innerhalb von 16 Basen liegt;
die zweite Mismatch-Base, liegt sie vor, des kompetitiven Primers 7 Basen oder mehr von dem 3'-Ende zur 5'-Seite positioniert ist.

## Revendications

1. Procédé de détection d'une séquence de base cible ayant une base polymorphe, comprenant
(a) une étape d'addition des amorces suivantes à un échantillon d'acide nucléique ayant un acide nucléique cible qui comprend une séquence de base incluant la séquence de base cible:
au moins un type d'amorce de détection qui est complémentaire de ladite séquence de base cible,
au moins un type d'amorce compétitive qui est complémentaire de ladite séquence de base cible tout en étant capable d'hybridation à un acide nucléique cible, d'une manière compétitive contre ladite amorce de détection, et
au moins un type d'amorce commune,
(b) une étape d'hybridation de ladite amorce de détection et de ladite amorce compétitive audit acide nucléique cible d'une manière compétitive avec l'utilisation de la séquence de base cible ayant la base polymorphe dans ledit échantillon d'acide nucléique comme modèle, entraînant de là une réaction d'extension pour synthétiser un produit d'extension A,
(c) une étape d'hybridation de ladite amorce commune audit produit d'extension A obtenu dans ladite étape (b) ou dans l'étape suivante (d), entraînant de là une réaction d'extension pour synthétiser un produit d'extension B,
(d) une étape d'hybridation de ladite amorce de détection ou de ladite amorce compétitive au produit d'extension B obtenu dans ladite étape (c), synthétisant de là le produit d'extension A, et
(e) une étape de détection du produit d'extension A ou du produit d'extension B, où
ladite amorce de détection possède une base d'appariement qui est complémentaire de la base polymorphe de la séquence cible, et possède au moins une première base de mésappariement qui n'est pas complémentaire d'une base de la séquence cible autre que la base polymorphe;
ladite amorce compétitive possède une base de mésappariement qui n'est pas complémentaire de la base polymorphe de la séquence cible, et qui possède au moins une première base de mésappariement et éventuellement au moins une seconde base de mésappariement qui ne sont pas complémentaires d'une base de la séquence cible autre que la base polymorphe;
la position de la au moins une première base de mésappariement de ladite amorce de détection est différente de la position de la au moins une première base de mésappariement de ladite amorce compétitive qui n'est pas complémentaire de la base de la séquence cible autre que la base polymorphe;
l'amorce de détection possède la base d'appariement qui est complémentaire de la base polymorphe de la séquence cible, à l'extrémité 3' ou la seconde base depuis l'extrémité 3';
ladite amorce commune est capable de constituer une paire d'amorces avec ladite amorce de détection ou ladite amorce compétitive pour amplifier ledit acide nucléique cible;
une différence dans une longueur de chaîne entre ladite amorce compétitive et ladite amorce de détection se situe à l'intérieur de 16 bases;
lorsqu'elle est présente, la seconde base de mésappariement de ladite amorce compétitive est localisée à 7 bases ou plus à distance de l'extrémité 3' vers l'extrémité 5'.

2. Procédé de détection d'une séquence de base cible selon la revendication 1, dans lequel
dans ladite amorce de détection et ladite amorce compétitive,
la première base de mésappariement qui n'est pas complémentaire d'une base de la séquence cible autre que la base polymorphe est localisée dans les 17 bases depuis la base d'appariement qui est complémentaire de la base polymorphe de la séquence cible dans le cas de ladite amorce de détection, et depuis la base de mésappariement qui n'est pas complémentaire de la base polymorphe de la séquence cible dans le cas de ladite amorce compétitive, et la position de chaque base de mésappariement de chaque amorce est différente.

3. Procédé de détection d'une séquence de base cible selon la revendication 1 ou 2, dans lequel ladite amorce de détection possède en outre au moins une seconde base de mésappariement qui n'est pas complémentaire d'une base de la séquence cible autre que la base polymorphe,
ladite amorce compétitive possède la seconde base de mésappariement,
la première base de mésappariement de l'amorce de détection est localisée dans les 6 bases depuis l'extrémité 3',
la seconde base de mésappariement de l'amorce de détection est localisée à 7 bases ou plus à distance depuis l'extrémité 3' vers l'extrémité 5', et
la seconde base de mésappariement de ladite amorce de détection et la seconde base de mésappariement de ladite amorce compétitive sont différentes l'une de l'autre.

4. Procédé de détection d'une séquence de base cible selon la revendication 3, dans lequel la position de ladite seconde base de mésappariement est la même pour à la fois ladite amorce de détection et ladite amorce compétitive.

5. Procédé de détection d'une séquence de base cible selon l'une quelconque des revendications 1 à 4, dans lequel lesdites étapes (b) à (d) sont des étapes exécutées par l'une quelconque sélectionnée parmi un groupe constitué de la réaction en chaîne de la polymérase, l'amplification isotherme à médiation en boucle, l'amplification à base de séquence d'acides nucléiques, l'amplification isotherme et chimère initiée par une amorce d'acides nucléiques, la réaction de transcription inverse-transcription concertée, l'amplification par déplacement de brin, l'amplification à médiation de transcription, du procédé Smart-amplification, de l'amplification par la recombinase polymérase, et de l'amplification dépendante de l'hélicase.

6. Procédé de détection d'une séquence de base cible selon l'une quelconque des revendications 1 à 5, dans lequel au moins l'une de ladite amorce de détection, ladite amorce compétitive, et ladite amorce commune est marquée.

7. Procédé de détection d'une séquence de base cible selon la revendication 6, dans lequel une substance de marquage pour l'utilisation dans ledit marquage est au moins l'un sélectionné dans un groupe constitué d'un fluorophore et d'un matériau d'absorption d'énergie.

8. Procédé de détection d'une séquence de base cible selon la revendication 6 ou la revendication 7, dans lequel ladite amorce de détection et ladite amorce compétitive sont respectivement marquées avec différents types de substances de marquage, et ladite étape (e) est une étape de détection séparément du produit d'extension de ladite amorce de détection et dudit produit d'extension de ladite amorce compétitive.

9. Procédé de détection d'une séquence de base cible selon l'une quelconque des revendications 6 à 8, dans lequel ladite étape (e) est une étape à exécuter simultanément auxdites étapes (b) à (d), tout comme étant une étape de détection d'un état dans lequel le produit d'extension à partir d'une amorce marquée forme un double brin.

10. Procédé de détection d'une séquence de base cible selon l'une quelconque des revendications 6 à 8, dans lequel ladite étape (e) est une étape à exécuter après ladite étape (d), tout en étant une étape d'exécution de la détection avec l'utilisation d'une courbe de fusion ou d'une courbe d'amplification dudit produit d'extension.

11. Procédé de détection d'une séquence de base cible selon l'une quelconque des revendications 6 à 10, dans lequel ladite étape (e) est une étape d'exécution de la détection à travers l'utilisation d'un procédé QP (sonde/amorce d'extinction).

12. Trousse pour l'utilisation dans le procédé de détection d'une séquence de base cible possédant une base polymorphe, comprenant au moins un type d'amorce de détection qui est complémentaire de ladite séquence de base cible,
au moins un type d'amorce compétitive qui est complémentaire de ladite séquence de base cible tout en étant capable d'hybridation à un acide nucléique cible d'une manière compétitive par rapport à ladite amorce de détection, et
au moins un type d'amorce commune, où
ladite amorce de détection possède une base d'appariement qui est complémentaire de la base polymorphe de la séquence cible, et possède au moins une première base de mésappariement qui n'est pas complémentaire d'une base de la séquence cible autre que la base polymorphe de ladite séquence cible;
ladite amorce compétitive possède une base de mésappariement qui n'est pas complémentaire de la base polymorphe de la séquence cible, et possède au moins une première base de mésappariement et éventuellement au moins une seconde base de mésappariement qui ne sont pas complémentaires d'une base de la séquence cible autre que la base polymorphe;
la position de la au moins une première base de mésappariement de ladite amorce de détection est différente de la position de la au moins une première base de mésappariement de ladite amorce compétitive qui n'est pas complémentaire de la base de la séquence cible autre que la base polymorphe;
l'amorce de détection possède la base d'appariement qui est complémentaire de la base polymorphe de la séquence cible, à l'extrémité 3' ou la seconde base à partir de l'extrémité 3';
ladite amorce commune est capable de constituer une paire d'amorces avec ladite amorce de détection ou ladite amorce compétitive pour amplifier ledit acide nucléique cible;
une différence dans une longueur de chaîne entre ladite amorce compétitive et ladite amorce de détection se situe dans les 16 bases;
lorsqu'elle est présente, la seconde base de mésappariement de ladite amorce compétitive est située à 7 bases ou plus à distance de l'extrémité 3' vers l'extrémité 5'.


### SEQUENCE LISTING

<110> TOPPAN Printing Co., Ltd.
<120> Method for identifying target nucleic acid sequence by competitive
   primer
<130> PC-13905
<150> JP 2010-68490
   <151> 2010-03-24
<160> 45
<170> PatentIn version 3.1
<210> 1
   <211> 139
   <212> DNA
   <213> Homo sapiens
<400> 1
   acctcccatc ctagtccaag ggtcgatgat ctcctggcac cgggcacctt tggccacgtc    60
   aggattccat gtcactgacc ctatcctccc ctctccccag accaggcccg gacgtggcta   120
   ctccgtaggc cctgctttt                                                139
<210> 2
   <211> 139
   <212> DNA
   <213> Homo sapiens
<400> 2
   acctcccatc ctagtccaag agtcgatgat ctcctggcac cgggcacctt tggccacgtc    60
   aggattccat gtcactgacc ctatcctccc ctctccccag accaggcccg gacgtggcta   120
   ctccgtaggc cctgctttt                                                139
<210> 3
   <211> 22
   <212> DNA
   <213> Artificial Sequence
<220>
   <223> Description of Artificial Sequence: VK1Wat-Acridine primer.
<400> 3
   tacctcccat cctagtccaa tg 22
<210> 4
   <211> 21
   <212> DNA
   <213> Artificial Sequence
<220>
   <223> Description of Artificial Sequence: VK1Mtg-Acridine primer.
<400> 4
   acctcccatc ctagtccatg a 21
<210> 5
   <211> 21
   <212> DNA
   <213> Artificial Sequence
<220>
   <223> Description of Artificial Sequence: VK1Mat primer.
<400> 5
   acctcccatc ctagtccaat a 21
<210> 6
   <211> 21
   <212> DNA
   <213> Artificial Sequence
<220>
   <223> Description of Artificial Sequence: VK1Mac primer.
<400> 6
   acctcccatc ctagtccaac a 21
<210> 7
   <211> 21
   <212> DNA
   <213> Artificial Sequence
<220>
   <223> Description of Artificial Sequence: VK1Mtg primer.
<400> 7
   acctcccatc ctagtccatg a 21
<210> 8
   <211> 21
   <212> DNA
   <213> Artificial Sequence
<220>
   <223> Description of Artificial Sequence: VK1Wat primer.
<400> 8
   acctcccatc ctagtccaat g 21
<210> 9
   <211> 16
   <212> DNA
   <213> Artificial Sequence
<220>
   <223> Description of Artificial Sequence: VK1R2 primer.
<400> 9
   aaaagcaggg cctacg 16
<210> 10
   <211> 22
   <212> DNA
   <213> Artificial Sequence
<220>
   <223> Description of Artificial Sequence: VK1Wat-pyrene primer.
<400> 10
   tacctcccat cctagtccaa tg 22
<210> 11
   <211> 22
   <212> DNA
   <213> Artificial Sequence
<220>
   <223> Description of Artificial Sequence: VK1Mtg-pyrene primer.
<400> 11
   tacctcccat cctagtccat ga 22
<210> 12
   <211> 21
   <212> DNA
   <213> Artificial Sequence
<220>
   <223> Description of Artificial Sequence: VK1M4t primer.
<400> 12
   acctcccatc ctagtcctag a 21
<210> 13
   <211> 21
   <212> DNA
   <213> Artificial Sequence
<220>
   <223> Description of Artificial Sequence: VK1M5t primer.
<400> 13
   acctcccatc ctagtctaag a 21
<210> 14
   <211> 21
   <212> DNA
   <213> Artificial Sequence
<220>
   <223> Description of Artificial Sequence: VK1M8t primer.
<400> 14
   acctcccatc ctattccaag a 21
<210> 15
   <211> 21
   <212> DNA
   <213> Artificial Sequence
<220>
   <223> Description of Artificial Sequence: VK1M10a primer.
<400> 15
   acctcccatc caagtccaag a 21
<210> 16
   <211> 21
   <212> DNA
   <213> Artificial Sequence
<220>
   <223> Description of Artificial Sequence: VK1M17t primer.
<400> 16
   accttccatc ctagtccaag a 21
<210> 17
   <211> 21
   <212> DNA
   <213> Artificial Sequence
<220>
   <223> Description of Artificial Sequence: VK1W4t primer.
<400> 17
   acctcccatc ctagtcctag g 21
<210> 18
   <211> 21
   <212> DNA
   <213> Artificial Sequence
<220>
   <223> Description of Artificial Sequence: VK1W5t primer.
<400> 18
   acctcccatc ctagtctaag g 21
<210> 19
   <211> 21
   <212> DNA
   <213> Artificial Sequence
<220>
   <223> Description of Artificial Sequence: VK1W8t primer.
<400> 19
   acctcccatc ctattccaag g 21
<210> 20
   <211> 21
   <212> DNA
   <213> Artificial Sequence
<220>
   <223> Description of Artificial Sequence: VK1W10a primer.
<400> 20
   acctcccatc caagtccaag g 21
<210> 21
   <211> 21
   <212> DNA
   <213> Artificial Sequence
<220>
   <223> Description of Artificial Sequence: VK1W17t primer.
<400> 21
   accttccatc ctagtccaag g 21
<210> 22
   <211> 23
   <212> DNA
   <213> Artificial Sequence
<220>
   <223> Description of Artificial Sequence: VK1Wat-P-FAM1 primer.
<400> 22
   cgacctccca tcctagtcca atg 23
<210> 23
   <211> 23
   <212> DNA
   <213> Artificial Sequence
<220>
   <223> Description of Artificial Sequence: VK1Mtg-23 primer.
<400> 23
   cgacctccca tcctagtcca tga 23
<210> 24
   <211> 24
   <212> DNA
   <213> Artificial Sequence
<220>
   <223> Description of Artificial Sequence: VK1Mtg-24 primer.
<400> 24
   ccgacctccc atcctagtcc atga 24
<210> 25
   <211> 25
   <212> DNA
   <213> Artificial Sequence
<220>
   <223> Description of Artificial Sequence: VK1Mtg-25 primer.
<400> 25
   cccgacctcc catcctagtc catga 25
<210> 26
   <211> 26
   <212> DNA
   <213> Artificial Sequence
<220>
   <223> Description of Artificial Sequence: VK1Mtg-26 primer.
<400> 26
   ccccgacctc ccatcctagt ccatga 26
<210> 27
   <211> 27
   <212> DNA
   <213> Artificial Sequence
<220>
   <223> Description of Artificial Sequence: VK1Mtg-27 primer.
<400> 27
   tccccgacct cccatcctag tccatga 27
<210> 28
   <211> 31
   <212> DNA
   <213> Artificial Sequence
<220>
   <223> Description of Artificial Sequence: VK1Mtg-31 primer.
<400> 28
   ctgttccccg acctcccatc ctagtccatg a 31
<210> 29
   <211> 35
   <212> DNA
   <213> Artificial Sequence
<220>
   <223> Description of Artificial Sequence: VK1Mtg-35 primer.
<400> 29
   tcctctgttc cccgacctcc catcctagtc catga 35
<210> 30
   <211> 43
   <212> DNA
   <213> Artificial Sequence
<220>
   <223> Description of Artificial Sequence: VK1Mtg-43 primer.
<400> 30
   ctgggctatc ctctgttccc cgacctccca tcctagtcca tga 43
<210> 31
   <211> 23
   <212> DNA
   <213> Artificial Sequence
<220>
   <223> Description of Artificial Sequence: VK1Wat-P-FAM2 primer.
<400> 31
   cgacctccca ttctagtcca atg 23
<210> 32
   <211> 23
   <212> DNA
   <213> Artificial Sequence
<220>
   <223> Description of Artificial Sequence: VK1Wat-P-FAM3 primer.
<400> 32
   cgacctcccg tcctagtcca atg 23
<210> 33
   <211> 23
   <212> DNA
   <213> Artificial Sequence
<220>
   <223> Description of Artificial Sequence: VK1Wat-P-FAM4 primer.
<400> 33
   cgacctctca tcctagtcca atg 23
<210> 34
   <211> 23
   <212> DNA
   <213> Artificial Sequence
<220>
   <223> Description of Artificial Sequence: VK1Wat-P-FAM5 primer.
<400> 34
   cgacttccca tcctagtcca atg 23
<210> 35
   <211> 23
   <212> DNA
   <213> Artificial Sequence
<220>
   <223> Description of Artificial Sequence: VK1Wat-P-FAM6 primer.
<400> 35
   ctacctccca tcctagtcca atg 23
<210> 36
   <211> 23
   <212> DNA
   <213> Artificial Sequence
<220>
   <223> Description of Artificial Sequence: VK1Wat-P-FAM7 primer.
<400> 36
   cgacctccca tcccagtcca atg 23
<210> 37
   <211> 23
   <212> DNA
   <213> Artificial Sequence
<220>
   <223> Description of Artificial Sequence: VK1Wat-P-FAM8 primer.
<400> 37
   cgacctccca tcctattcca atg 23
<210> 38
   <211> 23
   <212> DNA
   <213> Artificial Sequence
<220>
   <223> Description of Artificial Sequence: VK1Mtg-23-1 primer.
<400> 38
   cgacctccca tcctagtcca tga 23
<210> 39
   <211> 23
   <212> DNA
   <213> Artificial Sequence
<220>
   <223> Description of Artificial Sequence: VK1Mtg-23-2 primer.
<400> 39
   cgacctccca tactagtcca tga 23
<210> 40
   <211> 23
   <212> DNA
   <213> Artificial Sequence
<220>
   <223> Description of Artificial Sequence: VK1Mtg-23-3 primer.
<400> 40
   cgacctcccc tcctagtcca tga 23
<210> 41
   <211> 23
   <212> DNA
   <213> Artificial Sequence
<220>
   <223> Description of Artificial Sequence: VK1Mtg-23-4 primer.
<400> 41
   cgacctcaca tcctagtcca tga 23
<210> 42
   <211> 23
   <212> DNA
   <213> Artificial Sequence
<220>
   <223> Description of Artificial Sequence: VK1Mtg-23-5 primer.
<400> 42
   cgacatccca tcctagtcca tga 23
<210> 43
   <211> 23
   <212> DNA
   <213> Artificial Sequence
<220>
   <223> Description of Artificial Sequence: VK1Mtg-23-6 primer.
<400> 43
   caacctccca tcctagtcca tga 23
<210> 44
   <211> 23
   <212> DNA
   <213> Artificial Sequence
<220>
   <223> Description of Artificial Sequence: VK1Mtg-23-7 primer.
<400> 44
   cgacctccca tccgagtcca tga 23
<210> 45
   <211> 23
   <212> DNA
   <213> Artificial Sequence
<220>
   <223> Description of Artificial Sequence: VK1Mtg-23-8 primer.
<400> 45
   cgacctccca tcctaatcca tga 23